(19) Europäisches Patentamt
European Patent Office
Office européen des brevets

(11) EP 1 938 795 A2

(12) # EUROPEAN PATENT APPLICATION

(43) Date of publication:
**02.07.2008 Bulletin 2008/27**

(21) Application number: **07022955.4**

(22) Date of filing: **27.11.2007**

(51) Int Cl.:
***A61K 8/64*** $^{(2006.01)}$ ***A61K 8/65*** $^{(2006.01)}$
***A61K 8/73*** $^{(2006.01)}$ ***A61K 8/86*** $^{(2006.01)}$
***A61Q 5/00*** $^{(2006.01)}$ ***A61Q 5/04*** $^{(2006.01)}$
***A61K 8/04*** $^{(2006.01)}$

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **20.12.2006 KR 20060130568**
**09.05.2007 KR 20070045253**

(71) Applicant: **Sunbio, Inc.**
**Dongan-gu, Anyang-si**
**Gyeonggi-do 431-060 (KR)**

(72) Inventors:
- **Kwang, Nho**
  **Orinda**
  **CA 94563 (US)**
- **Hyun, Chang Ming**
  **Anyang-si**
  **Gyeonggi-do 431-723 (KR)**
- **Bae, Gun Won**
  **Seocho-gu**
  **Seoul 137-306 (KR)**
- **Yoon, So Jeong**
  **Gwanak-gu**
  **Seoul 151-767 (KR)**
- **Ahn, Min Jung**
  **Yongin-si**
  **Gyeonggi-do 446-913 (KR)**

(74) Representative: **Scheele, Friedrich et al**
**Stolmár Scheele & Partner**
**Blumenstrasse 17**
**80331 München (DE)**

(54) **Hair treatment composition, hair treatment agent and method for treating hair by using the same**

(57) The present invention relates to a hair treatment composition comprising a multi-arm polyethylene glycol derivative (A) containing two or more functional groups that may be covalently bound to amine, a hair treatment composition comprising a polyethylene glycol derivative (B), containing one or more functional groups that may react with the functional groups of said composition, and/or a biocompatible polymer (C), a hair treatment agent comprising these compositions and a method for using the same. The hair treatment agent of the present invention is capable of increasing the hair thickness and volume, restoring the damaged hair and lasting the hair shape and curls for a long time, by firstly covalently binding some functional groups of said polyethylene glycol derivative (A) to amino acids present on hair surfaces and then covalently binding some functional groups not covalently bound to hair, to a polyethylene glycol derivative (B) and/or a biocompatible polymer (C).

Fig. 1

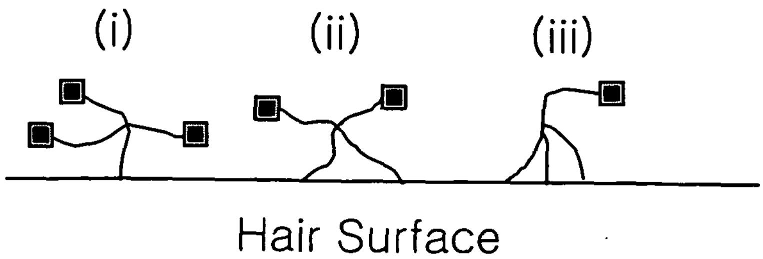


EP 1 938 795 A2

## Description

This application claims the benefit of the filing date of Korean Patent Application No. 10-2006-0130568 filed on December, 20, 2006 and Korean Patent Application No. 10-2007-0045253 filed on May 9, 2007 in the Korean Intellectual Property Office, the disclosure of which is incorporated herein in its entirety by reference.

## FIELD OF THE INVENTION

The present invention relates to a hair treatment composition comprising a multi-arm polyethylene glycol derivative (A) containing two or more functional groups that may be covalently bound to amines, a hair treatment composition comprising a polyethylene glycol derivative (B), containing one or more functional groups that may react with the functional groups of said composition, and/or a biocompatible polymer (C), a hair treatment agent comprising these compositions and a method for using the same. Specifically, the present invention relates to a hair treatment composition being capable of increasing the hair thickness and volume, restoring the damaged hair and lasting the hair shape and curls for a long time, by firstly covalently binding some functional groups of said polyethylene glycol derivative (A) to amino acids present on hair surfaces and then covalently binding some functional groups not covalently bound to hair, to a polyethylene glycol derivative (B) and/or a biocompatible polymer(C); a hair treatment agent thereof; and a method for treating hair using the same.

## BACKGROUND OF THE INVENTION

Keratin, a major component of hair, consists of 18 amino acids. Particularly, in the human keratin, cystine occupies the highest ratio as 16%, and among amino acids, glutamic acid, arginine, and lysine occupy 14.8%, 9.6% and 2.6%, respectively. In general, bonds for maintaining hair shape are ionic bonds between cationic amino acids such as lysine or arginine and anionic amino acids such as asphartic acid or glutamic acid; or covalent bonds such as disulfide bridge (-S-S-) between two molecules of cysteine. In addition, hydrogen bonds, polar bonds, or non-covalent bonds such as London force are present as intermolecular attracting forces. The number of hair of a person is about 100,000 ~ 150,000, and its growing rate is 0.2 ~ 0.5 mm/day.

The feature of hair also varies with races. Average hair thickness of Caucasoid is 55 μm, whereas that of Negroid and Mongoloid is 72 μm. Therefore, hair of Caucasoid is thinner by about 25%. In addition, hair of Negroid makes an oval form as the ratio of the major axis to the minor axis is 1.75, while hair of Caucasoid and Mongoloid is close to a circle as the ratio is 1.25 ~ 1.35.

Meanwhile, hair is fallen out in the Talogen stage (resting stage) of hair growth stages, and the amount of hair loss at this time corresponds to 4 to 14% of total hair. Hair loss may be promoted by strong brushing under the resting stage. Postpartum or post-menopause women have higher speed of hair loss. An average of more than 50-100 strands of hair may be fallen out in the resting stage. A symptom of hair loss is that the hair thickness is thinning. Hair is thinning with age, and thus hair loss is caused.

Although such hair loss may be genetically developed, it may be caused by usually lacking interest and care of hair. Especially, it is recently noted that hair loss by stress is also increased in the younger generation. Therefore, person being anxious about hair loss uses any method such as using a wig or a toupee, adhering other processed hair by adhesives, or pinning hair pieces to abundantly increase hair volume. In addition, a method of increasing hair volume is used, such as changing hair style or using a hair gel, a mousse or a fixing spray. However, said methods have disadvantages that they are temporary and have to be cared each time.

In researches using polymers as hair care products, several precedents are found. US Patent No. 6,447,803 to Sorrentino, et al. discloses that polymers being hydrophobic and alkali-soluble and polysaccharides such as xanthan gum, or surfactants such as polyalkylene glycol and boric acid are together added and the resulting products are used as hair care products. Monomers of said polymers have any structure that carboxylic acids are bound to carbon atoms with double bonds of alpha and beta types. Also, alkyl acrylates or methacrylates may be bound to the carboxylic acids. In this patent, it is described that hair may be well elongated, have good feeling and elasticity without tangle and have any thick feeling, on applying polymers, including such acrylate/methyacrylate esters, to hair as a hair gel.

US 7,048,916, and US 6,548,051 to Rollat, et al., and Garnier, et al., respectively, disclosed that there was an effect to keep a stylish hair, on applying copolymers of methacrylates and acrylates bound to branched or linear alkyl alcohols or cyclic alcohols to hair. US 6,410,005 to Gallengullos, et al. disclosed that copolymers, in which a hydrophilic acrylate structure was bound to a hydrophobic polyacrylate backbone, provided hair with flowability, style retention at high humidity and volume sense and prevented from drooping of curls. US 6,436,412 to Quinn introduced a method for coating polymers containing at least functional groups capable of forming hydrogen bonds with metal cations, to keratin surfaces, and described that the polymers might be used as hair styling agents, besides mascaras and lipsticks. US 6,258,347 to Sakuta, et al. mentioned that films were formed on hair surfaces, using silicon polymers, to afford an

excellent glossiness regardless of an amount of moisture in air.

Said references disclose that the effects of stylishness, moisturizing or volume sense may be improved by forming particles or films on hair surfaces. However, the products obtained from said processes are washed out on shampooing hair, so that the effect is not sustained and is temporary. Hair cosmetic materials must be covalently bound to hair, to show the sustained effect.

## SUMMARY OF THE INVENTION

Accordingly, these inventors have accomplished the present invention by confirming that when polyethylene glycol hydrogels are formed on hair using a hair treatment formulation comprising a hair treatment composition comprising a multi-arm polyethylene glycol derivative (A) containing two or more functional groups that may be covalently bound to amines; and a hair treatment composition comprising a polyethylene glycol derivative (B), containing one or more functional groups that may react with the functional groups of said composition, and/or a biocompatible polymer (C), hair thickness is increased, hair strength is strong felt and curl persistency is increased.

Therefore, the object of the present invention is to provide a hair treatment composition comprising a multi-arm polyethylene glycol derivative (A) containing two or more functional groups that may be covalently bound to amines. Another object of the present invention is to provide a hair treatment composition comprising a polyethylene glycol derivative (B), containing one or more functional groups that may react with the functional groups of said composition, and/or a biocompatible polymer (C).

The other object of the present invention is to provide a hair treatment agent comprising said hair treatment compositions and a method for treating hair using the same.

To achieve these objects, the present invention provides a hair treatment composition comprising a multi-arm polyethylene glycol derivative (A) containing two or more functional groups that may be covalently bound to amines (in some cases, referred to 'hair treatment composition A,' below), a hair treatment composition comprising a polyethylene glycol derivative (B), containing one or more functional groups that may react with the functional groups of said composition, and/or a biocompatible polymer (C) (in some cases, referred to 'hair treatment composition B,' below), a hair treatment agent comprising the compositions A and B, and a method for treating hair using the hair treatment agent.

The present invention is characterized in that the hair treatment composition A containing two or more functional groups that may be covalently bound to amines is used to improve the ability of binding between hair and amines.

The present invention has the other characteristic that hydrogels may be formed on hair, by treating hair with a hair treatment composition A and further with a hair treatment composition B, to increase hair thickness and volume, restore the damaged hair, and last hair shape and curls for a long time.

## BRIEF DESCRIPTION OF DRAWINGS

Fig. 1 is a conceptual view representing that 4-arm PEG-ASG is bound to lysine of hair, on hair surfaces, according to one embodiment of the present invention.

Fig. 2 is a conceptual view representing that a PEG-AM derivative or a biocompatible polymer is bound to 4-arm PEG-ASG bound on hair surfaces, according to another embodiment of the present invention.

Fig. 3 is a photograph depicting the SDS-PAGE result of analyzing polyethylene glycol hydrogels.

Fig. 4 is a photograph depicting the SDS-PAGE result of analyzing hydrogels formed from polyethylene glycol-serum protein.

Fig. 5 is a photograph depicting the SDS-PAGE result of analyzing hydrogels formed from polyethylene glycol-chitosan.

Fig. 6 is a photograph depicting the SDS-PAGE result of analyzing hydrogels formed from polyethylene glycol-phytocollagen.

Fig. 7 is a photograph depicting the SDS-PAGE result with a concentration of 4-arm PEG-ASG on hair surface.

Fig. 8 is a photograph depicting the SDS-PAGE result with a hair reaction time of 4-arm PEG-ASG on hair surface.

Fig. 9 is a photograph depicting the SDS-PAGE result with a reaction pH of 4-arm PEG-ASG on hair surface.

Fig. 10 is a photograph depicting the SDS-PAGE result with a reaction buffer solution of 4-arm PEG-ASG on hair surface.

Fig. 11 is a photograph depicting the SDS-PAGE result showing yields of extracting polyethylene glycol covalently bound to hair with extraction solutions.

Fig. 12 is a photograph depicting the SDS-PAGE result showing formation of polyethylene glycol hydrogels with a reaction time of 4-arm PEG-ASG.

Fig. 13 is a photograph depicting the SDS-PAGE result showing formation of polyethylene glycol hydrogels in at

least 1% 6-arm polyethylene glycol-amine solution.

Fig. 14 is a photograph depicting the SDS-PAGE result showing formation of polyethylene glycol hydrogels in at least 5% 6-arm polyethylene glycol-amine solution.

Fig. 15 is a photograph depicting the SDS-PAGE result showing formation of polyethylene glycol hydrogels with a reaction time of 6-arm polyethylene glycol-amine.

Fig. 16 is a photograph depicting the SDS-PAGE result showing formation of polyethylene glycol hydrogels on measuring cross section of hair.

Fig. 17 is a graph depicting increase of cross section in hair on which polyethylene glycol hydrogels are formed.

Fig. 18 is a photograph depicting the SEM result of analyzing hair on which polyethylene glycol hydrogels are formed.

Fig. 19 is a photograph depicting the SDS-PAGE result showing the repeated effect in forming polyethylene glycol hydrogels.

Fig. 20 is a photograph depicting the SDS-PAGE result showing persistency of polyethylene glycol hydrogels on hair surfaces.

Fig. 21 is a photograph depicting the test results comparing curl persistency with addition of dimethicone in polyethylene glycol hydrogel compositions.

Fig. 22 is a photograph depicting the test results comparing curl persistency with addition of glycerin in polyethylene glycol hydrogel compositions.

## DETAILED DESCRIPTION OF PREFERRED EMBODIMENTS

The present invention is described in more detail below.

The present invention relates to a hair treatment composition (hair treatment composition A) comprising a multi-arm polyethylene glycol derivative (A) containing two or more functional groups that may be covalently bound to amines.

It is preferred that said polyethylene glycol derivative (A) has one or more functional groups capable of covalently binding to amines at each branch. As shown in Figs. 1 and 2, the polyethylene glycol derivative (A) with such a structure has not only a high probability that may be covalently bound to amines of hair, but also the remaining functional groups that are not covalently bound to amines of hair can be bound to further coating layers and/or functional layers.

It is preferred that the polyethylene glycol derivative (A) includes two or more units of Formula 1

$$[(OCH_2CH_2)_n\text{-P-Q-R-S}] \qquad 1$$

in which

n represents 10 ~ 10,000,

P represents a single bond, an oxygen atom, a sulfur atom or X-Y, wherein X represents a sulfur atom, an oxygen atom or NH, and Y represents carbonyl (C=O), carbonyloxy (COO) or CONHCO,

Q represents an alkylene group having 1 to 8 carbon atoms,

R represents carbonyloxy (COO), and

S represents a succineimindyl group.

Said unit of Formula 1 is, preferably, one or more selected from the group consisting of polyethylene glycol succinimidyl glutarate (PEG-SG), polyethylene glycol succinimidyl succinate (PEG-SS), polyethylene glycol succinimidyl adipate (PEG-SA), polyethylene glycol succinimidyl pimelate (PEG-SP), polyethylene glycol amide-succinimidyl succinate (PEG-ASS), polyethylene glycol amide-succinimidyl glutarate (PEG-ASG), polyethylene glycol amide-succinimidyl adipate (PEG-ASA), polyethylene glycol amide-succinimidyl pimelate (PES-ASP), polyethylene glycol urethane-succinimidyl succinate (PEG-UTSS), polyethylene glycol urethane-succinimidyl glutarate (PEG-UTSG), polyethylene glycol urethane-succinimidyl adipate (PEG-UTSA), polyethylene glycol urethane-succinimidyl pimelate (PES-UTSP), polyethylene glycol urea-succinimidyl succinate (PEG-USS), polyethylene glycol urea-succinimidyl glutarate (PEG-USG), polyethylene glycol urea-succinimidyl adipate (PEG-USA), polyethylene glycol urea-succinimidyl pimelate (PEG-USP), polyethylene glycol thio-succinimidyl succinate (PEG-TSS), polyethylene glycol thio-succinimidyl glutarate (PEG-TSG), polyethylene glycol thio-succinimidyl adipate (PEG-TSA) and polyethylene glycol thio-succinimidyl pimelate (PES-TSP), and more preferably, polyethylene glycol amide succinimidyl glutarate (PEG-ASG).

Specific examples of said units having Formula 1 may be each represented by the following formula:

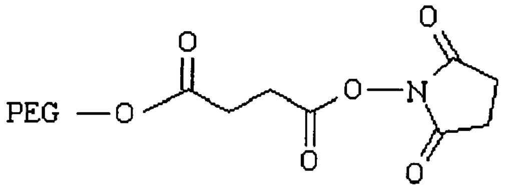

(PEG-SS)

2a

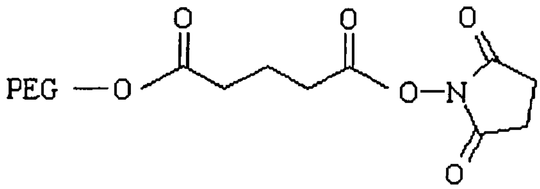

(PEG-SG)

2b

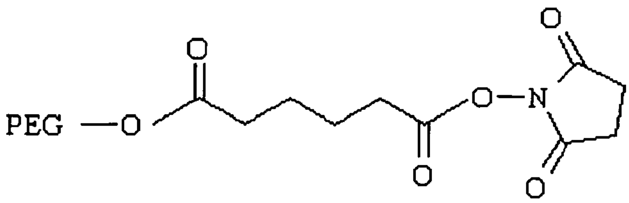

(PEG-SA)

2c

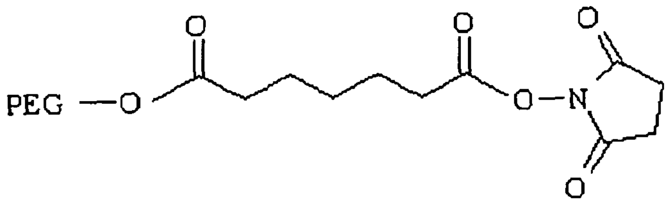

(PEG-SP)

2d

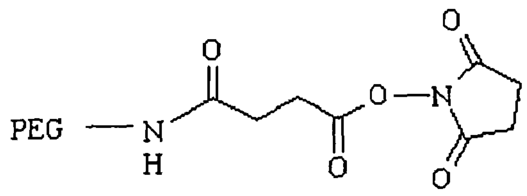

(PEG-ASS)

3a

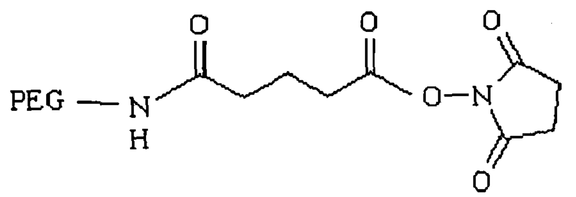

(PEG-ASG)

3b

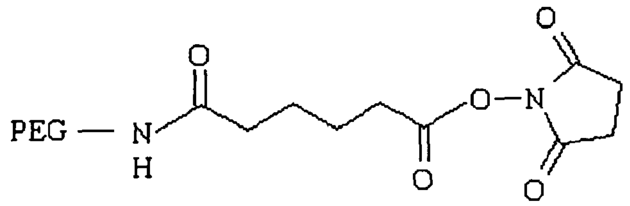

(PEG-ASA)

3c

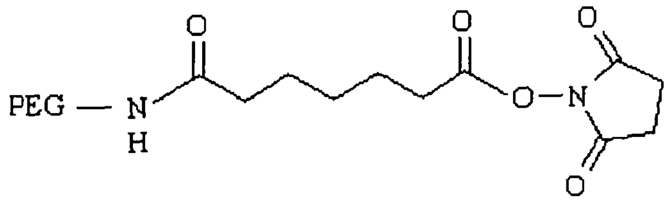

(PEG-ASP)

3d

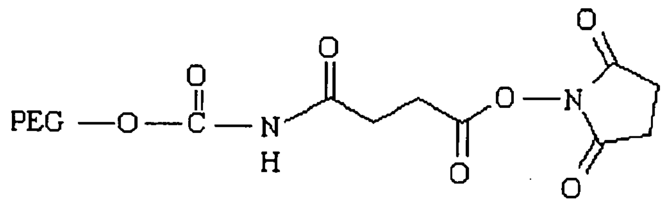

(PEG-UTSS)

4a

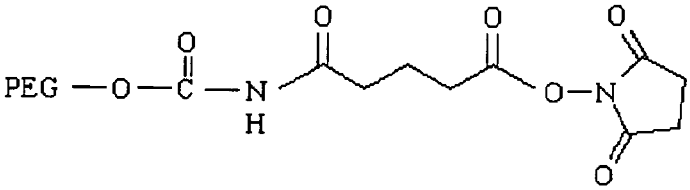

(PEG-UTSG)

4b

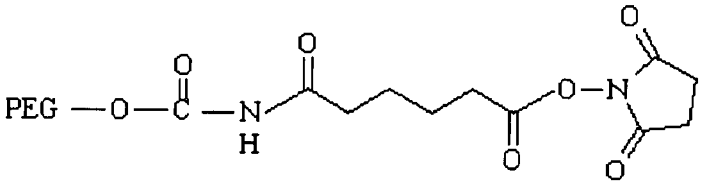

(PEG-UTSA)

4c

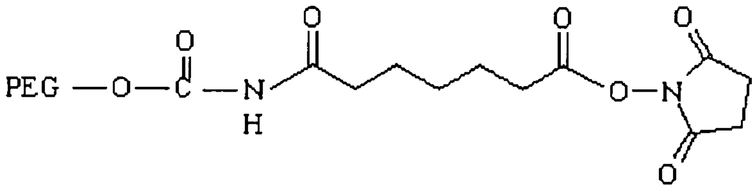

(PEG-UTSP)

4d

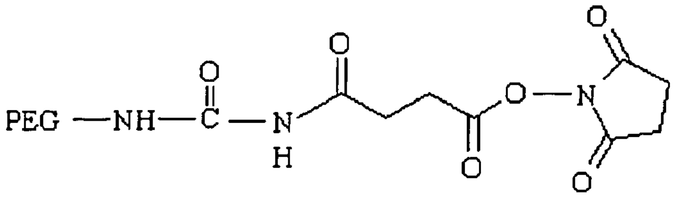

(PEG-USS)

5a

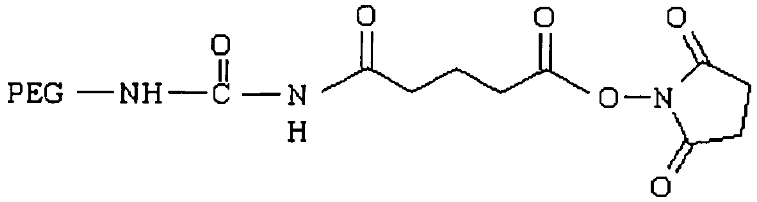

(PEG-USG)

5b

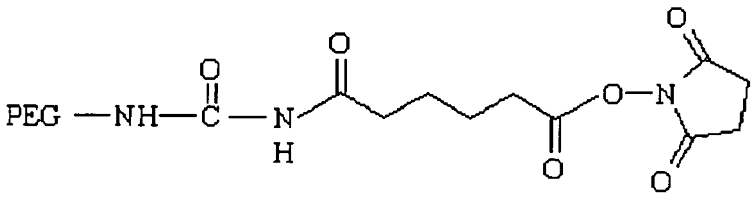

(PEG-USA)

5c

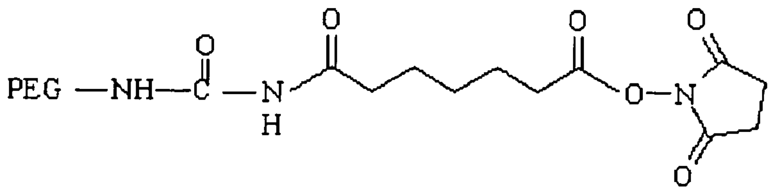

(PEG-USP)

5d

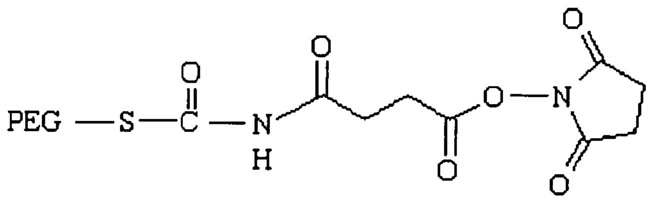

(PEG-TSS)

6a

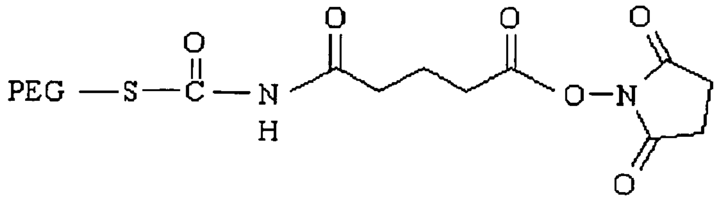

(PEG-TSG)

6b

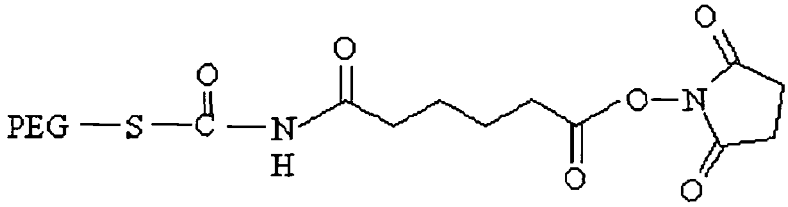

(PEG-TSA)

6c

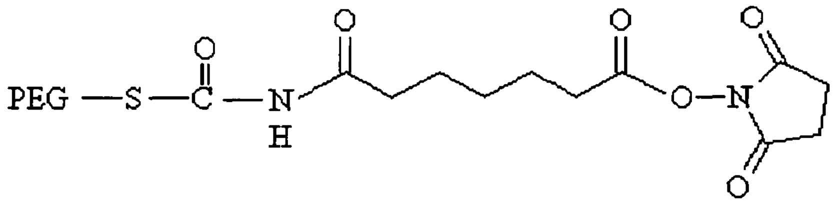

(PEG-TSP)

6d

As long as the PEG derivative (A) used herein has a multi-arm structure, it may be used without limitation. Preferably, it has 2-arm, 3-arm, 4-arm, 6-arm or 8-arm structure, and more preferably, 4-arm structure, in consideration of the reaction efficiency. When the present PEG derivative (A) has at least 3-arm structure, the derivative (A) has, preferably, a structure including a core. Said core may include, but not limited to, glycerols or saccharides. For example, the 2-arm, 4-arm and 6-arm PEG derivative (A) containing said unit of Formula 1 include the compounds of Formulas 7, 8 and 9, respectively.

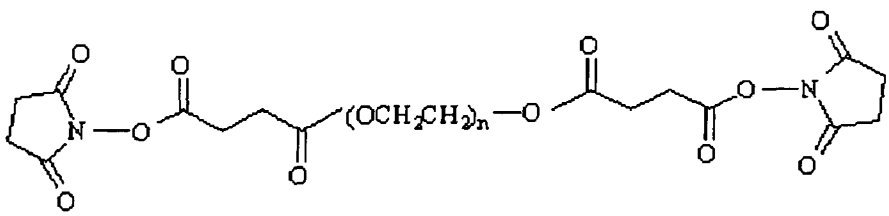

7

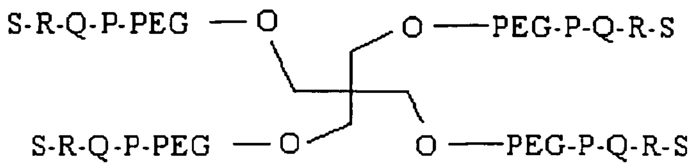

8

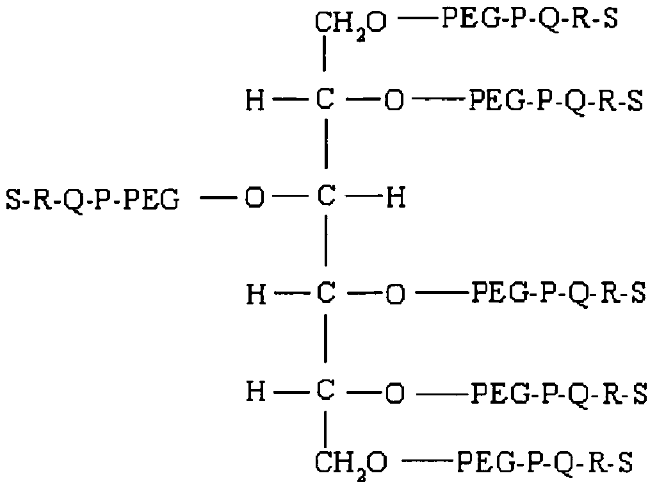

9

In said PEG derivative (A), each PEG having various molecular weights, preferably a molecular weight of 1,000 to 1,000,000 daltons, may be used without limitation. If the molecular weight is less than 1,000 daltons, the PEG derivative may show toxicity. In addition, as the molecular weight is increased, the interference on binding the PEG derivative to hair is caused, so that the covalent bonds to hair may be inhibited. Therefore, if the molecular weight is in excess of 1,000,000 daltons, the reaction bond of the PEG derivative to hair may be considerably lowered.

In addition, the content of said PEG derivative (A) is, preferably 1 to 30% w/v, relative to the composition of total hair treatment agent. If the amount is less than 1% w/v, the PEG derivative has to be bound to the restricted amino acids containing amines present on hair surfaces, so that the yield of covalent bonds is lowered and the excellent efficacy cannot be expected. If the amount is in excess of 30% w/v, the efficiency may be lowered, since the amino acids containing amines present on hair are restricted, despite of increase in the amount of covalent bonds.

The present invention also relates to a hair treatment composition comprising one or more selected from the group consisting of a polyethylene glycol derivative (B) and a biocompatible polymer (C), wherein the polyethylene glycol derivative (B) and the biocompatible polymer (C) contain one or more functional groups that may react with the functional groups of said polyethylene glycol derivative (A).

In the polyethylene glycol derivative (B) or the biocompatible polymer (C), the functional groups that may react with the functional groups of said polyethylene glycol derivative (A) are not specifically limited, but may include amines as a preferred example.

As shown in Fig. 2, the polyethylene glycol derivative (B) or the biocompatible polymer (C) may react with the functional groups of polyethylene glycol derivative (A) to form hydrogels, followed by thickening hair thickness or contributing various functions. In addition, when the polyethylene glycol derivative (B) or the biocompatible polymer (C) used herein is selected from those having multi-arm structures as in the polyethylene glycol derivative (A), the polyethylene glycol derivative (A) may also again react with the formed hydrogels above to form the repeated hydrogels.

Preferably, the polyethylene glycol derivative (B) used herein is a multi-arm PEG-AM including two or more units of Formula 10.

$[(OCH_2CH_2)_n\text{-}NH_2]$ 10

in which n is 10 ~ 10,000.

The structure of said polyethylene glycol derivative (B) includes, but not specifically limited to, a linear, 2-arm, 3-arm, 4-arm, 6-arm or 8-arm structure. Considering the functionality described above and the repeated formation possibility and reactivity, it has, preferably, at least 3-arm structure, and more preferably 6-arm structure.

In said PEG derivative (B), each PEG having various molecular weights, preferably a molecular weight of 1,000 to 1,000,000 daltons, may be used. If the molecular weight is less than 1,000 daltons, the PEG derivative may show toxicity. In addition, as the molecular weight is increased, the interference on binding the PEG derivative to hair is caused, so that the covalent bond to hair may be inhibited. Therefore, if the molecular weight is in excess of 1,000,000 daltons, the reaction bond of the PEG derivative with hair may be considerably lowered.

In addition, the content of said PEG derivative (B) is, preferably 1 to 30% w/v, relative to the composition of total hair treatment agent. If the amount is less than 1% w/v, the PEG derivative has to be bound to the restricted amino acids containing amines present on hair surfaces, so that the yield of covalent bonds is lowered and the excellent efficacy cannot be expected. If the amount is in excess of 30% w/v, the efficiency may be lowered, since the amino acids containing amines present on hair are restricted, despite of increase in the amount of covalent bonds.

Examples of the biocompatible polymer (C) used herein include one or more selected from the group consisting of serum proteins, chitosan, phytocollagen, keratin, elastin, peptides and polypeptides. Preferably, one or more selected from the group consisting of serum protein, chitosan, and polypeptides may be used.

More specifically, the biocompatible polymer (C) used herein includes serum protein; proteins, including vegetable or animal proteins, having two or more amines; deacetylated chitosan having two or more amines and a molecular weight of 1,000 to 1,000,000 daltons, preferably 1,000 to 200,000 daltons; animal or vegetable polypeptides such as keratin, elastin, bean or barley as a material with molecular weight reduced by an enzyme, pH and the like; or hydrolysates, and the like. Theses polymers may contribute to functionality by reacting with functional groups of polyethylene glycol derivative (A) to form hydrogels.

The hair treatment compositions of the present invention may further include usual additives well known in this field. These additives are specifically illustrated below, but are not limited or restricted to the examples.

Specifically, it is preferred that the hair treatment composition of the present invention further includes viscosity modifiers or emulsifiers. The viscosity modifier or emulsifier includes one or more selected from Natrosol 100, lecithin, N-methylpyrrolidone (EG, NMP), dimethicon, and glycerin. Natrosol 100 may be added for regulating the viscosity, N-methylpyrrolidone may increase PEGylation of hair and dimethicone or glycerin may give moisturizing property or oily sense to hair. The term 'PEGgylation' used herein means that a polyethylene glycol derivative (A) forms covalent bonds with hair proteins; or a polyethylene glycol derivative (B) or a biocompatible polymer (C), to be adhered. Each component above may be used by mixing with the hair treatment composition A; or the hair treatment composition B of the present invention, whose amount may be determined by optimizing each concentration of components.

The reaction scheme 1 below depicts a reaction scheme that PEG amide succinimidyl glutarate (PEG-ASG), an example of unit included in the polyethylene glycol derivative (A) of the present invention is covalently bound to hair keratin.

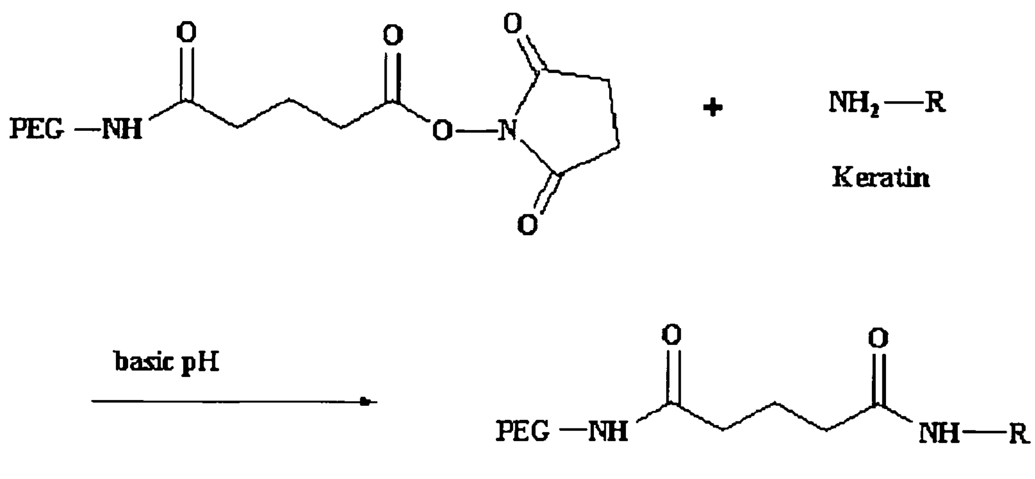


Reaction Scheme 1

As represented in the reaction scheme 1 above, PEG-ASG is so electophilic that it may form covalent bonds with nucleophilic functional groups having unshared pairs such as $-NH_2$, -OH and -SH. An amide linker present between polyethylene glycol and succinimidyl glutarate gives stability to the entire structure after covalent binding. When the amide linker is not present, an ester bond between polyethylene glycol backbone and succinimidyl glutarate may be hydrolyzed to lose activity of the derivative in water solution. To increase the bond efficiency and persistency of polyethylene glycol derivative (A), it is preferred that linkers such as amide, urethane, urea, or thio are present within the derivative.

As described above, the amount of lysine in hair is only 2.6%. Cysteine in hair is almost present as cystine, which has a weak hydrophilic property. To bind to arginine, the reaction should be performed at more than pH 10. Therefore, the hair treatment composition of the present invention is preferably bound to an amine of lysine at pH 6.5 to 10, in consideration of pH 4.5 ~ 10 appropriate to use cosmetics. Preferably, the hair treatment composition of the present invention has a pH of 6.5 to 10, and more preferably a pH of 8.5 to 9.5. If pH is less than 6.5, the yield of PEGylation in the polyethylene glycol derivative is lowered. If pH is in excess of 10, hair may result in damage.

The present invention also relates to a hair treatment agent comprising

a hair treatment composition comprising a multi-arm polyethylene glycol derivative (A) containing two or more functional groups that may be covalently bound to amines; and

a hair treatment composition comprising a polyethylene glycol derivative (B), containing one or more functional groups that may react with the functional groups of said derivative (A), and/or a biocompatible polymer (C).

In case of using the hair treatment agent of the present invention, the hair thickness and volume may be increased, the damaged hair may be restored and the hair shape and curls may be lasted for a long time by firstly forming the primary layer on hair with the hair treatment composition A and optionally forming the secondary layer on the primary layer with the hair treatment composition B. Preferably, the hair treatment agent of the present invention used herein includes, but not limited to, a form of kit wherein the hair treatment composition A and the hair treatment composition B are included in a separate 2 pack type. Of course, said compositions may be each separately used.

The present invention also relates to a method for treating hair which comprises a step of applying to hair a composition comprising a multi-arm polyethylene glycol derivative (A), containing two or more functional groups that may be covalently bound to amines, to covalently bind the polyethylene glycol derivative (A) to hair.

Preferably, the hair treating method according to the present invention further comprises a step of applying a composition comprising a polyethylene glycol derivative (B) having functional groups that may react with functional groups of the polyethylene glycol derivative (A), or a biocompatible polymer (C) to form hydrogels.

The present invention is described, based on drawings, in more detail below.

In Fig. 1, a form that 4-arm polyethylene glycol derivative (A) containing units of PEG-ASG may be bound to lysine of hair on hair surfaces is depicted. Probability of binding 4-arm polyethylene glycol derivative (A) to lysine of hair surfaces is four times as high as that of one-arm methoxy PEG-ASG. Once one amide succinimidyl glutarate is bound to an amine of lysine (i), the possibility of binding the remaining three functional groups to the nearby lysine is more increased (ii, iii). As not shown in the drawing, it was also confirmed that all four amide succinimidyl glutarate is each bound to lysine could be present.

In addition, Fig. 2 is a view representing the state of binding a functional material, including polyethylene glycol derivative (B), chitosan containing a deacetylated amine group, proteins such as keratin or elastin, a hydrolyzed low molecular weight polypeptide, phytokeratin, and the like, to the bound 4-arm polyethylene glycol derivative (A) to hair surfaces. The polyethylene glycol derivative (B) with amines as a terminal group was depicted as an example in this figure. Methoxy polyethylene glycol amine (mPEG-AM) has one amine, 2-arm polyethylene glycol amine (2-arm PEG-AM) two amines, 4-arm polyethylene glycol amine (4-arm PEG-AM) four amines and 6-arm polyethylene glycol amine (6-arm PEG-AM) six amines.

In addition, proteins such as keratin and elastin, or chitosan as well as multi-arm polyethylene glycol amines may include several amines per one molecule or one polymer. Especially, chitosan may include tens to hundreds per polymer, depending on the degree of deacetylation.

As shown in Fig. 2, the bonds of 4-arm polyethylene glycol derivative (A) on hair surfaces may form the primary layer thereon. The more the damaged hair by perm, bleaching, or dyeing were, the more the covalent bonds of 4-arm polyethylene glycol derivative were increased. This is considered to be due to large exposure of lysine present in hair, as hair is damaged. That is, the covalent bonds keeping hair are damaged by physical or chemical hair treatments and then peptide linkages of proteins constituting hair are broken to flow small peptides or amino acids out. Consequently, amino acids exposing on hair surfaces may be increased. In addition, when 4-arm polyethylene glycol derivative (A) was covalently bound, it could be confirmed that moisturizing property of hair, curl persistency, volume sense on rinsing and the like were improved.

In Fig. 2, the primary layer consists of 4-arm polyethylene glycol derivative (A) including units of PEG-ASG covalently bound to at least one position on hair. The intact functional groups of amide succinimidyl glutarate having activity, may be present in the primary layer, to which polyethylene glycol derivative (B) including amines, etc., proteins

such as chitosan, keratin or elastin, hydrolyzed low molecular polypeptide or phytokeratin may be secondarily bound. Such materials comprising amine functional groups form the secondary layer. When the primary layer and the secondary layer are cross-linked, hydrogels may be formed.

The more multi-arm the polyethylene glycol derivative has or the higher its concentration is, the more easily the polyethylene glycol hydrogels may be formed. In its high concentration, "visual polyethylene glycol hydrogels" can be confirmed. As used herein, the term "visual polyethylene glycol hydrogels" means a form of rigidly harden solid gels that may see with eyes. It is not desirable to coat hair with such visual polyethylene glycol hydrogels. In the present invention, it is preferred to form hydrogels with a level of nanometers to micrometers. The formed polyethylene glycol hydrogels are covalently bound to hair. In addition, functional materials, including polyethylene glycol derivative (B) containing amines participated in polyethylene glycol hydrogels, keep not only functionality as such, but are also linked to hair by covalent bonds. Therefore, the functionality may be kept for a long time. Furthermore, it is confirmed that since the polyethylene glycol hydrogels are formed on the hair surfaces, the effect of increasing the hair thickness as much as thickness of hydrogels may be obtained.

All the added amine functional groups are not bound to polyethylene glycol hydrogels once formed on the hair surfaces. The amine functional groups not participated in polyethylene glycol hydrogels may be present. These groups may be regulated by changing a molar ratio of functional groups, for example, amide succinimidyl glutarate , in the primary layer to amines in the secondary layer. When the intact amine functional groups are again treated with the described 4-arm polyethylene glycol derivative (A), they are donated to the derivative (3rd layer). Therefore, following forming hydrogels, a significant number of amines may be increased, although the amine number is restricted in hair itself.

## EXAMPLES

The present invention is explained as examples and experimental examples in detail below. However, the examples and experimental examples are intended to illustrate the present invention, and do not restrict the scope of the present invention.

### Example 1: Formation of polyethylene glycol hydrogels

5% w/v 4-arm polyethylene glycol derivative containing polyethylene glycol amide succinimidyl glutarate (referred to '4-arm PEG-ASG,' below, manufactured by SUNBIO INC. (Korea), P4ASG-20) (MW 20,000 daltons) was dissolved in 20 mM phosphate buffer with pH 9 to prepare a solution. 4-arm PEG-AM (MW 20,000 daltons) solution with concentrations described in Table 1 below was separately prepared in the same buffer solution. Two solutions were mixed in the same volume (500 $\mu\ell$) and allowed to stand for 40 minutes. The results were represented in the following Table 1.

Table 1

| Concentration (w/v) of 4-arm PEG-AM | 1% | 1.5% | 2% | 3% | 4% | 5% |
|---|---|---|---|---|---|---|
| Visual PEG hydrogels | - | - | - | + | + | + |
| +: Visual PEG hydrogels are observed.<br>-: Visual PEG hydrogels are not observed. | | | | | | |

As shown in Table 1 above, when the concentration of 4-arm PEG-AM is 3% or more, visual PEG hydrogels were appeared. In case of 2% PEG-AM, a significant viscosity was appeared over 1% and 1.5% PEG-AM, but visual hydrogels were not formed. In the concentration of 1.5% or less, visual PEG hydrogels could not yet be appeared. The solutions without appearing formation of visual hydrogels were subjected to SDS polyacrylamide electrophoresis (SDS-PAGE) and then Titrisol® stain analysis. Specifically, SDS was removed from SDS-PAGE gel surfaces subjected to electrophoresis with distilled water, and the gel was soaked in 5% barium chloride solution for 5 minutes. Again, its surfaces were washed with distilled water. Since the repeat unit, $-CH_2CH_2O-$, of PEG has a property of chelating metal ions, barium ions are chelated to PEG. Gel was washed with distilled water to remove barium chloride adhered to gel surfaces. The resulting gel was soaked in the appropriately diluted Titrisol solution for 5 minutes and washed with distilled water. Iodines in the Titrisol solution were bound to barium cations chelated to PEG to represent the positions in red color. The results were represented in Fig. 3. As shown in Fig. 3, it could be confirmed that high molecular weight hydrogels were formed in the concentrations of 1% and 1.5%. The formation of PEG hydrogels depended on each concentration of PEG and pH of buffer, and was increased as the reaction time lasted long. In the solution mixed with 2% 4-arm PEG-AM for a long time, it could be confirmed that visual hydrogels were formed over time.

**Example 2: Formation of hydrogels in polyethylene glycol mouse serum protein**

3% w/v 4-arm PEG-ASG (MW 20,000 daltons) was dissolved in 20 mM phosphate buffer with pH 9 to prepare a solution. Mouse serum protein solution with concentrations of 0.03 to 0.5% was separately prepared in the same buffer solution. Two solutions were mixed in the same volume (500 $\mu\ell$) and allowed to stand for 40 minutes. Hydrogels of polyethylene glycol-serum protein analyzed by the same method as Example 1 were represented in Fig. 4.
As shown in Fig. 4, the formation of visual hydrogels could not be confirmed in mouse serum protein with low concentration, but the formation of hydrogels having a size of less than micrometers was confirmed by SDS-PAGE analysis.

**Example 3: Formation of hydrogels in polyethylene glycol-chitosan**

3% w/v 4-arm PEG-ASG (MW 20,000 daltons) was dissolved in 20 mM phosphate buffer with pH 9 to prepare a solution. Chitosan (MW 200,000 daltons) solution (pH 5.0) with concentrations of 0.015 to 2% was separately prepared in the same buffer solution. Two solutions were mixed in the same volume (500 $\mu\ell$) and allowed to stand for 40 minutes. The resulting product was subjected to SDS-PAGE analysis and Titrisol® stain analysis, and the formed hydrogels of polyethylene glycol-chitosan were represented in Fig. 5. When the concentration of chitosan solution was 1% or more, it could be confirmed that visual hydrogels were formed.

**Example 4: Formation of hydrogels in polyethylene glycol-phytocollagen**

3% w/v 4-arm PEG-ASG (MW 20,000 daltons) was dissolved in 20 mM phosphate buffer with pH 9 to prepare a solution. A solution adding phytocollagen with concentrations of 0.3 to 5% was separately prepared in the same buffer solution. Two solutions were mixed in the same volume (500 $\mu\ell$) and allowed to stand for 40 minutes. The resulting product was subjected to SDS-PAGE analysis, and then Titrisol® stain analysis, the formed hydrogels of polyethylene glycol-phytocollagen were represented in Fig. 6.
As shown in Fig. 6, when the concentration of phytocollagen was 0.3% or more, it could be confirmed that hydrogels of polyethylene glycol-phytocollagen were formed.

**Example 5: Formation of hydrogels on hair**

**Pretreatment of hair**

The healthy hair obtained from a hair shop was bleached three times or subjected to perm procedures to induce some damage to be added thereto. The obtained bundle of hair was washed with 1.5% SLES (sodium lauryl ether sulfate) surfactant. With being sufficiently left at room temperature, it was dried and then used.

**PEGylation of the primary layer using the hair treatment composition A**

4-arm PEG-ASG (MW 20,000 daltons) was dissolved in 20 mM phosphate buffer with pH 9 or 20 mM carbonate with pH 9 to prepare the hair treatment composition A. Each bundle of hair was treated with this composition to form the primary layer.

**PEGylation of the secondary layer using the hair treatment composition B**

The bundle of hair treated in the example above was rinsed with distilled water, or absorbed with a paper towel to remove the remaining PEG solution after forming the primary layer. 6-arm PEG-AM (MW 10,000 daltons) was dissolved in 20 mM carbonate buffer solution to prepare the hair treatment composition B. This composition was evenly applied to the bundle of hair forming the primary layer, and reacted for 30 minutes. 4-arm PEG-ASG and 6-arm PEG-AM, which were covalently bound to at least one position of hair via the reaction, consisted of intermolecular lattices to form the secondary layer and then hydrogel layer together with the primary layer.
The prepared hair was sufficiently washed with 1.5 wt% SLES solution and distilled water to remove the unreacted PEG. The washed hair was completely dried at room temperature, and finely cut with scissors. Then, 0.2 g of hair pieces was soaked in 5 ml of distilled water and boiled at 80°C for breaking covalent bonds between hair and PEG. After boiling for 16 hours, PEG was precipitated in 1 ml or 2 ml of said solution, using TCA (TCA precipitation, trichloroacetic acid). After centrifugation, the precipitate was again dissolved using 0.1M NaOH and then analyzed by SDS-PAGE. For detecting PEG on SDS-PAGE gel, Titrisol® staining was used (Test method used in Example 1).

**Experimental Example 1: Covalent bond of PEG derivative (A) with hair**

Each 0.5 g of hair bundle (11 cm) was subjected to PEGylation in 4-arm PEG-ASG (MW 20,000 daltons) solution (20 mM phosphate buffer, pH 9.0) with each concentration of 2.5% and 5% w/v, for 40 minutes. Covalent bonds of 4-arm PEG derivative (A) with hair were represented in Fig. 7.

Fig. 7 shows that 4-arm PEG derivative is covalently bound to hair (arrow). When 4-arm PEG without any functional group was used, it was confirmed that following hair treatment, the derivative was washed out during hair rinse procedures. In addition, as the concentration of 4-arm PEG derivative (A) was higher, the amount subjected to PEGylation in hair was more increased. Hair not treated with 4-arm PEG derivative (A) was used as a control group. Bands were shifted upward on SDS-PAGE relative 4-arm PEG derivative (A). This was because water molecules were bound to the repeat units (-($CH_2CH_2O$)-) of PEG, showing increase of molecular weight.

**Experimental Example 2: Degree of PEGylation with reaction time**

Each 0.5 g of hair bundle (11 cm) was subjected to PEGylation in 5% w/v 4-arm PEG-ASG (MW 20,000 daltons) solution (20 mM phosphate buffer, pH 9.0) for 10, 20, 30 and 40 minutes. Yields of PEGylation with reaction times were represented in Fig. 8.

As shown in Fig. 8, degree of reacting 4-arm PEG derivative (A) with amine functional groups of hair was in proportion to the elapsed time. The reactivity of amide succinimidyl glutarate was initiated as dissolved in the buffer. Particularly, when the reaction time was 40 minutes, PEG was subjected to PEGylation in the most degree.

**Experimental Example 3: Degree of PEGylation with pH of buffers**

Each 0.5 g of hair bundle (11 cm) was subjected to PEGylation in 5% w/v 4-arm PEG-ASG (MW 20,000 daltons) solution (20 mM phosphate buffer, pH 9.0, 9.5, 10) for 40 minutes. Yields of PEGylation with reaction pH were represented in Fig. 9.

As shown in Fig. 9, the reaction of amide succinimidyl glutarate was well performed in an alkali pH. It could be confirmed that the reactivity was best at pH 9.5.

**Experimental Example 4: Degree of PEGylation with kinds of buffer**

Each 0.5 g of hair bundle (11 cm) was subjected to PEGylation in 5% w/v 4-arm PEG-ASG (MW 20,000 daltons) solution (20 mM phosphate buffer, pH 9.0, or 20 mM carbonate buffer, pH 9.5) for 40 minutes. Yields of PEGylation with kinds of buffer were represented in Fig. 10.

As shown in Fig. 10, the functional groups of amide succinimidyl glutarate were reacted with hair regardless of buffer kinds. It can be said that the reaction depends on pH or reaction times rather than buffer kinds. In the reaction of amide succinimidyl glutarate , there is a phenomenon that the pH is lowered, as the succinimidyl functional groups are reacted. It could be confirmed that the carbonate buffer (pKa2= 10.33) is buffered the reaction of amide succinimidyl glutarate (pH 9-10) more effectively than the phosphate buffer (pKa2=7.2, pKa3=12.43).

**Experimental Example 5: Yields of extraction with extracting solutions**

Each 0.5 g of hair bundle (11 cm) was subjected to PEGylation in 5% w/v 4-arm PEG-ASG (MW 20,000 daltons) solution (20 mM phosphate buffer, pH 9.0) for 40 minutes. To extract PEG bound to hair, the hair bundle was each soaked in distilled water, or 20 mM phosphate buffer solutions (pH 6.5, 7.5, 8.5), and then boiled at 80°C for 16 hours. Extraction yields of PEGylated hair with PEG extracting solutions were set forth in Fig. 11.

As shown in Fig. 11, the degrees of extracting PEG with pH of extracting solutions were similar to each other. It could be confirmed from these results that PEG extracted from hair is not bound by an electrostatic bond or a hydrogen bond.

**Experimental Example 6: Formation of PEG hydrogels with reaction times of 4-arm PEG-ASG**

Each 0.5 g of hair bundle (11 cm) was subjected to PEGylation in 5% w/v 4-arm PEG-ASG (Mw 20,000 daltons) solution (20 mM phosphate buffer, pH 9.0) for 15, 20, and 25 minutes. Following suitably removing the remaining solution on hair with a paper towel, the hair was treated with 5% w/v 6-arm PEG-AM solution (20 mM phosphate buffer, pH 9.0) for 30 minutes. The reason of removing the unreacted, remaining 4-arm PEG derivative on hair is because the remaining 4-arm PEG derivative visibly forms hydrogels on treating 6-arm PEG-AM solution (Mw 10,000). Therefore, the solution may form invisible nanometers-scale hydrogels together with 4-arm PEG derivative bound to hair surfaces and obtain

the effect of covalently binding to hair surfaces. As the control group (C), only 4-arm PEG-ASG (Mw 20,000) was reacted for 40 minutes. The primary layer of PEG hydrogels formed on hair surfaces by the process as above was set forth in Fig. 12.

As shown in Fig. 12, when only 4-arm PEG-ASG (Mw 20,000 daltons) as the control group (C) was reacted for 40 minutes, 20k PEG bends were most detected. This is because the degree of PEGylation is increased with reaction times of 4-arm PEG-ASG. When hair was treated with 6-arm PEG-AM instead, the degree of 20k bends was weak, but could be confirmed that 6-arm PEG-AM forms invisible nanometers-scale PEG hydrogels, in combination with 4-arm PEG-ASG bound to hair surfaces, to increase molecular weight.

**Experimental Example 7: Formation of PEG hydrogels with concentrations of PEG-AM**

0.5g of hair bundle (11 cm) was subjected to PEGylation in 5% w/v 4-arm PEG-ASG (Mw 20,000 daltons) solution (20 mM phosphate buffer, pH 9.0) for 40 minutes. Hair was light rinsed with distilled water, instead of using a paper towel, and treated with 6-arm PEG-AM solution (20 mM phosphate buffer, pH 9.0) having a concentration 1% or 2% w/v for 30 minutes. The formed secondary layer of PEG hydrogels on hair surfaces was set forth in Fig. 13.

As shown in Fig. 13, it could be confirmed that PEG hydrogels are produced in at least 1% 6-arm PEG-AM solution.

**Experimental Example 8: Formation of PEG hydrogels with concentrations of PEG-AM**

Each 0.5 g of hair bundle (11 cm) was subjected to PEGylation in 5% w/v 4-arm PEG-ASG (Mw 20,000 daltons) solution (20 mM phosphate buffer, pH 9.0) for 40 minutes. Following removing the remaining solution on hair with a paper towel, the hair was treated with 2%, 5%, or 10% w/v 6-arm PEG-AM (Mw 10,000) solution (20 mM phosphate buffer, pH 9.0) for 30 minutes. The formed PEG hydrogels were set forth in Fig. 14.

As shown in Fig. 14, when hair was treated with 6-arm polyethylene glycol amine solution, it could be seen to produce hydrgels. Particularly, it could be confirmed that a large quantity of hydrogels are formed in case of above 5%.

**Experimental Example 9: Formation of PEG hydrogels with reaction time of polyethylene glycol amine**

Each 0.5 g of hair bundle (11 cm) was subjected to PEGylation in 5% w/v 4-arm PEG-ASG (Mw 20,000 daltons) solution (20 mM phosphate buffer, pH 9.0) for 40 minutes. Following removing the remaining solution on hair with a paper towel, the hair was treated with 5% w/v 6-arm polyethylene glycol amine (Mw 10,000 daltons) solution (20 mM phosphate buffer, pH 9.0) for 10, 20, and 30 minutes. The formed PEG hydrogels were set forth in Fig. 15.

As shown in Fig. 15, when hair was treated with 6-arm polyethylene glycol amine solution for above 10 minutes, it could be confirmed that PEG hydrogels are formed.

**Experimental Example 10: Cross-section area of hair on which PEG hydrogels are formed**

To show whether hair thickness is increased by forming PEG hydrogels on hair surfaces, hair thickness was measured using Laser Scan Micrometer (LSM 3100, Mitutoyo). To measure hair thickness using Laser Scan Micrometer, hair was prepared as follows: One strand of 3 cm long hair may be bitten at both ends by a bite and placed on a track of Laser Scan Micrometer to measure the hair thickness. Following measuring thickness (exactly cross-section area) prior to and after PEGylation of the same hair, the increased thickness was calculated. Samples of hair were measured by 20 strands, and the mean value was calculated. When each sample of hair was subjected to PEGylation, the hair bundle was subjected to PEGylation together to confirm whether PEG hydrogels were formed in SDS-PAGE.

Specimens for electrophoresis used herein were prepared as follows: each 0.5 g of hair bundle (11 cm) was subjected to PEGylation in 5% w/v 4-arm PEG-ASG (Mw 20,000 daltons) solution (20 mM phosphate buffer, pH 9.0) for 30 minutes. Following light removing the remaining solution on hair with a towel, the hair was treated with 5% w/v 6-arm PEG-AM (Mw 10,000 daltons) solution (20 mM phosphate buffer, pH 9.0) for 30 minutes (4ASG-6AM). In addition, as a negative control group, only buffers, in which PEG derivative was removed in each PEG derivative solution, were treated for the same time during the above procedure (Buffer). As a positive control group, 5% w/v 4-arm PEG-ASG was treated for 40 minutes (4ASG). The formed PEG hydrogels and the increase rate of hair cross-section area were set forth in Figs. 16 and 17.

As shown in Figs. 16 and 17, when the hair was treated with only buffers (60 minutes), the hair thickness was rather reduced. It is possible that matrices or small-sized amino acids flow out from hair during treatment of hair in the buffer for 60 minutes. When the hair was treated with 4-arm PEG-ASG only, the cross-section area of hair was increased by 1.45%. It could be confirmed that when the hair was sequentially treated with 4-arm PEG-ASG and 6-arm PEG-AM, the cross-section area of hair was increased by 7.64%.

**Experimental Example 11: SEM analysis of hair on which PEG hydrogels are formed**

SEM analysis was performed using samples (0.5 cm) of hair in Experimental Example 10. Each hair strand was ion-coated with Pt or Pd, and the hair surfaces were observed using scanning electron microscope (Hitach, S4700). The results were set forth in Fig. 18.

On analyzing the results, the surfaces of A and B in Fig. 18, treated with buffer and 4-arm PEG-ASG, respectively, are not different from those of hair without any treatment, as shown in Fig. 18. However, in case of C in Fig. 18, wherein the hair was treated with 4-arm PEG-ASG and then 6-arm PEG-AM, PEG hydrogels can be observed on the hair surfaces. PEG hydrogels with an average diameter of 60 nm can be seen on the particle phase plate, and even the overlapped phase plates (arrow). In an organoleptic aspect, no difference of softness was on treating with buffer and 4-arm PEG-ASG. However, when PEG hydrogels were formed as in C, softness was disappeared and stiffness was strengthened. Instead, it could be confirmed that the strength of hair is high.

**Experimental Example 12: Effect of repeating PEG hydrogels**

Each 0.5 g of hair bundle (11 cm) was subjected to PEGylation in 5% w/v 4-arm PEG-ASG (Mw 20,000 daltons) solution (20 mM phosphate buffer, pH 9.5) for 30 minutes. Following light removing the remaining solution on hair with a towel, the hair was treated with 5% w/v 6-arm PEG-AM (Mw 10,000 daltons) solution (20 mM phosphate buffer, pH 9.5) for 30 minutes. The above procedure was repeated twice and three times. After the procedure was completed each time, hair was rinsed clearly. As a control group, the hair was treated with 5% 4-arm PEG-ASG only. The results were set forth in Fig. 19.

As a result, when the hair was treated with 4-arm PEG-ASG only, high molecular weight PEG hydrogels were not formed, as shown in Fig. 19. However, when the hair was treated with 4-arm PEG-ASG and further 6-arm PEG-AM, PEG hydrogels were faintly formed on the well of electrophoresis. When the once PEGylated hair was subjected to the second and third PEGylation, the increased PEG hydrogels could be seen. However, the band corresponding to molecular weight of 20,000 on SDS-PAE electrophoresis of 4-arm PEG-ASG (Mw 20,000) could be seen to be maintained in the same amount in all samples. Such results mean that the binding sites of 4-arm PEG-ASG on hair surfaces are restricted. It could be confirmed that the increased PEG hydrogels might be PEG hydrogels further bound on the firstly formed PEG hydrogels.

**Experimental Example 13: Persistency of PEG hydrogels on hair surfaces**

Each 0.5 g of hair bundle (11 cm) was subjected to PEGylation in 5% w/v 4-arm PEG-ASG (Mw 20,000 daltons) solution (20 mM phosphate buffer, pH 9.5) for 30 minutes. Following light removing the remaining solution on hair with a towel, the hair was treated with 5% w/v 6-arm PEG-AM (Mw 10,000 daltons) solution (20 mM phosphate buffer, pH 9.5) for 30 minutes. After the procedure was completed, hair was 30 times rinsed with 1.5% SLES solution. The results were set forth in Fig. 20.

To confirm whether PEG hydrogels last in hair, it was rinsed using a surfactant. As shown in Fig. 20, the amount of hydrogels was reduced with increasing rinse times. But, it was confirmed that when the hair was rinsed by even 30 times, hydrogels were remained. It could be confirmed that the PEG hydrogels of the present invention may contribute to hair persistent effects rather than temporary effects.

**Experimental Example 14: Measurement of hair curl persistency using a composition of PEG hydrogels**

**<u>Preperation of a hair treatment composition</u>**

**(1) Hair treatment composition comprising PEG derivative (A)**

20 mM carbonate buffer solution (sodium bicarbonate 1.68 g/L distilled water) with a pH of 9.5 was prepared. 50 to 60 ml of the buffer solution was added in a beaker. The other components except for PEG derivative (A) were added to the solution to prepare a composition. The added components were sufficiently dissolved. Then, PEG derivative (A) (4-arm PEG-ASG, available from SUNBIO, Inc.) was added to the solution and dissolved, with shaking the mixture. The resulting solution was titrated by 100 ml, using 20 mM carbonate buffer solution, to obtain a composition. The amounts of each component added to the composition are shown in Table 2 below

Table 2

| | Control | A-1 | A-2 | A-3 | A-4 | A-5 | A-6 | A-7 |
|---|---|---|---|---|---|---|---|---|
| PEG derivative (A) | | 5 g | 5 g | 5 g | 5 g | 5 g | 5 g | 5 g |
| Natrosol 100 | | | 0.6 g | 0.6 g | 0.6 g | 0.6 g | 0.6 g | 0.6 g |
| N-methylpyrrolidone | | | 0.5 ml | 0.5 ml | 0.5 ml | 0.5 ml | 0.5 ml | 0.5 ml |
| Dimethicone | | | 1 g | | | | | |
| Lecithin | | | 1 g | | | | | |
| Marin elastin | | | | | | 5 ml | | 5 ml |
| Glycerin | | | | | | 4 ml | | 4 ml |

**(2) Hair treatment composition comprising PEG derivative (B)**

The hair treatment composition (B) was prepared by the same method as (1) above, except that 6-arm PEG amine as PEG derivative (B) was used instead of PEG derivative (A). The amounts of each component are shown in Table 3 below.

Table 3

| | Control | B-1 | B-2 | B-3 | B-4 | B-5 | B-6 | B-7 |
|---|---|---|---|---|---|---|---|---|
| PEG derivative (B) | | 5 g | 5 g | 5 g | 5 g | 5 g | 5 g | 5 g |
| Natrosol 100 | | | 0.6 g | 0.6 g | 0.6 g | 0.6 g | 0.6 g | 0.6 g |
| N-methylpyrrolidone | | | 0.5 ml | 0.5 ml | 0.5 ml | 0.5 ml | 0.5 ml | 0.5 ml |
| Dimethicone | | | | 1 g | 1 g | | | |
| Lecithin | | | | 1 g | 1 g | | | |
| Marin elastin | | | | | | | 5 ml | 5 ml |
| Glycerin | | | | | | | 4 ml | 2 ml |

**Measurement of hair curl persistency**

Hydrogels were formed from the compositions prepared in Table 2 and 3 above by the same method as Example 5. To measure hair curl persistency of PEG hydrogel compositions, an experiment was performed by the following process.

The composition of Table 2 above was applied to hair and reacted at room temperature for 30 minutes. The remaining solution on hair was suitably removed with a towel. The composition of Table 3 was evenly applied to the treated hair in the first step and reacted at room temperature for 30 minutes. The hair was three times shampooed using 1.5% SLES surfactant to remove the remaining composition. Hair without any treatment was used as a control group (no treatment).

Hair (L0, 18 cm) was wound around Lot 10, allowed to stand at 40°C for 20 minutes, and removed from the Lot, for comparing curl persistency. The first length of total hair curls ($L_{t1}$) was measured, and the length of drooping hair after 17 hours ($L_{t2}$) was measured.

In addition, compositions A-1 to A-3 in Table 2 and compositions B-1 to B-3 in Table 3 were subjected to the same process, followed by measuring curl persistency of hair. Curl persistency of hair was calculated using Formula A below. The results were set forth in Table 4 to 5 and Figs. 21 to 22.

$$\text{Curl Persistency (\%)} = (L_0 - L_{t2}) / (L0 - L_{t1}) \times 100 \qquad \text{A}$$

($L_0$: initial length of hair (18 cm), $L_{t1}$: hair length measured immediately after removed from the Lot, $L_{t2}$: hair length measured 17 hours after removed from the Lot)

Table 4

| Hair length (cm) | No treatment (Control group) | Formulation | | |
|---|---|---|---|---|
| | | 1 (A-1/B-1) | 2 (A-2/B-2) | 3 (A-3/B-3) |
| Immediately ($L_{t1}$) | 2.8 | 3.0 | 3.0 | 3.0 |
| after 17 h ($L_{t2}$) | 7.3 | 6.8 | 6.4 | 6.2 |
| Curl persistency (%) | 70.4 | 74.7 | 77.3 | 78.7 |

As a result of experiment, it could be confirmed that when PEG hydrogels were covalently bound to hair, and dimethicone and lecithin were used as composition of formulations (Formulation 3 > Formulation 2 > Formulation 1), curl persistency was excellent, and that when the hair was touched, it was soft and had an excellent elasticity (refer to Fig. 21), as shown in Table 4 and Fig. 21.

Table 5

| Hair length (cm) | Hair (no treatment) | Formulation | | | |
|---|---|---|---|---|---|
| | | 4 (A-4/B-4) | 5 (A-5/B-5) | 6 (A-6B-6) | 7 (A-7/B-7) |
| Immediately ($L_{t1}$) | 2.5 | 2.5 | 2.8 | 2.8 | 2.7 |
| After 17h ($L_{t2}$) | 4.8 | 4.3 | 4.1 | 4.1 | 4.3 |
| Curl persistency (%) | 85.2 | 88.4 | 91.4 | 91.4 | 89.5 |

As a result of experiment, it could be confirmed that when PEG hydrogels were covalently bound to hair, and glycerin and marin elastin were used as composition of formulations, curl persistency was excellent over formulation (A-4/B-4) adding dimethicone as a major component, as shown in Table 5 and Fig. 22 (A-5/B-5, A-6/B-6 > A-7/B-7 > A-4/B-4). It could be seen that when glycerin was added (A-5/B-5, A-6/B-6, A-7/B-7), curl persistency and elasticity were good, and softness of hair was more increased.

Formulations of hair coating composition comprising PEG hydrogels of the present invention are explained below, with provision that the present invention is specifically explained, without limitation.

| **Formulation 1: Liquid formulation (1)** | |
|---|---|
| 4-arm polyethylene glycol succinimidyl glutarate | 5 g |
| Natrasol 100 | 0.6 g |
| N-methylpyrrolidone | 0.5 ml |
| ※ 20 mM carbonate buffer solution pH 9.5, as titrated by 100 mL | |

| **Formulation 2: Liquid formulation (2)** | |
|---|---|
| 6-arm polyethylene glycol amine | 5 g |
| Natrasol 100 | 0.6 g |
| N-methylpyrrolidone | 0.5 ml |
| Dimethicone | 1 g |
| Lecithin | 1 g |
| ※ 20 mM carbonate buffer solution pH 9.5, as titrated by 100 mL | |

## INDUSTRIAL APPLICABILITY

Hair treatment agents of the present invention are covalently bound to hair, and thus, may increase not only hair thickness and volume, restore the damaged hair, last hair shape and curls for a long time, but also contribute other functionalities.

## Claims

1. A hair treatment composition comprising a multi-arm polyethylene glycol derivative (A) containing two or more functional groups that may be covalently bound to amines.

2. The hair treatment composition of claim 1, **characterized in that** the polyethylene glycol derivative (A) includes two or more units of Formula 1:

$$[(OCH_2CH_2)_n\text{-P-Q-R-S}] \qquad 1$$

in which
n represents 10 ~ 10,000,
P represents a single bond, an oxygen atom, a sulfur atom or X-Y, wherein X represents a sulfur atom, an oxygen atom or NH, and Y represents carbonyl (C=O), carbonyloxy (COO) or CONHCO,
Q represents an alkylene group having 1 to 8 carbon atoms,
R represents carbonyloxy (COO), and
S represents a succinimidyl group.

3. The hair treatment composition of claim 2, **characterized in that** the unit of Formula 1 is one or more selected from the group consisting of polyethylene glycol succinimidyl glutarate (PEG-SG), polyethylene glycol succinimidyl succinate (PEG-SS), polyethylene glycol succinimidyl adipate (PEG-SA), polyethylene glycol succinimidyl pimelate (PEG-SP), polyethylene glycol amide-succinimidyl succinate (PEG-ASS), polyethylene glycol amide-succinimidyl glutarate (PEG-ASG), polyethylene glycol amide-succinimidyl adipate (PEG-ASA), polyethylene glycol amide-succinimidyl pimelate (PEG-ASP), polyethylene glycol urethane-succinimidyl succinate (PEG-UTSS), polyethylene glycol urethane-succinimidyl glutarate (PEG-UTSG), polyethylene glycol urethane-succinimidyl adipate (PEG-UTSA), polyethylene glycol urethane-succinimidyl pimelate (PES-UTSP), polyethylene glycol urea-succinimidyl succinate (PEG-USS), polyethylene glycol urea-succinimidyl glutarate (PEG-USG), polyethylene glycol urea-succinimidyl adipate (PEG-USA), polyethylene glycol urea-succinimidyl pimelate (PES-USP), polyethylene glycol thio-succinimidyl succinate (PEG-TSS), polyethylene glycol thio-succinimidyl glutarate (PEG-TSG), polyethylene glycol thio-succinimidyl adipate (PEG-TSA) and polyethylene glycol thio-succinimidyl pimelate (PES-TSP).

4. The hair treatment composition of claim 1, **characterized in that** the polyethylene glycol derivative (A) has a 2-arm, 3-arm, 4-arm, 6-arm or 8-arm structure.

5. The hair treatment composition of claim 1, **characterized in that** the polyethylene glycol derivative (A) has a molecular weight of 1,000 ~ 1,000,000 daltons.

6. The hair treatment composition of claim 1, **characterized in that** the content of polyethylene glycol derivative (A) is 1 ~ 30% w/v.

7. A hair treatment composition comprising one or more selected from the group consisting of a polyethylene glycol derivative (B) and a biocompatible polymer (C), in which is **characterized in that** the polyethylene glycol derivative (B) and the biocompatible polymer (C) include one or more functional groups that may react with functional groups of the polyethylene glycol derivative (A) of claim 1.

8. The hair treatment composition of claim 7, **characterized in that** the functional group is amine.

9. The hair treatment composition of claim 7, **characterized in that** the polyethylene glycol derivative (B) has a 2-arm, 3-arm, 4-arm, 6-arm or 8-arm structure.

10. The hair treatment composition of claim 7, **characterized in that** the polyethylene glycol derivative (B) has a

molecular weight of 1,000 ~ 1,000,000 daltons.

**11.** The hair treatment composition of claim 7, **characterized in that** the content of polyethylene glycol derivative (B) is 1 ~ 30% w/v.

**12.** The hair treatment composition of claim 7, the biocompatible polymer (C) is one or more selected from the group consisting of serum protein, chitosan, phytocollagen, keratin, elastin, peptide and polypeptide from animals and plants.

**13.** The hair treatment composition of claim 1 or 7, further comprising a viscosity modifier or an emulsifier.

**14.** The hair treatment composition of claim 1 or 7, **characterized in that** the pH is 6.5 ~ 10.

**15.** A hair treatment agent comprising a composition of claim 1 and a composition of claim 7.

**16.** A method for treating hair which comprises the step of
applying to hair a composition comprising a multi-arm polyethylene glycol derivative (A) containing two or more functional groups that may be covalently bound to amine to form covalent bonds of the polyethylene glycol derivative (A) thereto.

**17.** The method for treating hair of claim 16, which further comprises the step of applying a composition comprising one or more selected from the group consisting of a polyethylene glycol derivative (B) and a biocompatible polymer (C), containing one or more functional groups that may react with functional groups of the polyethylene glycol derivative (A), to form hydrogels.

Fig. 1

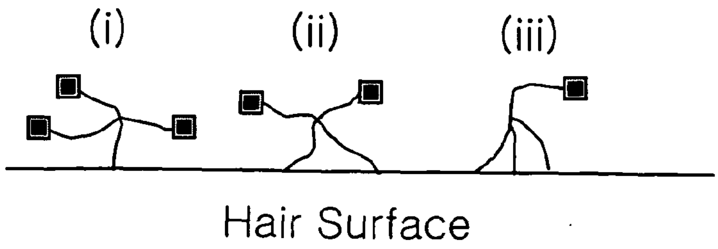
(i)
(ii)
(iii)
Hair Surface

Fig. 2

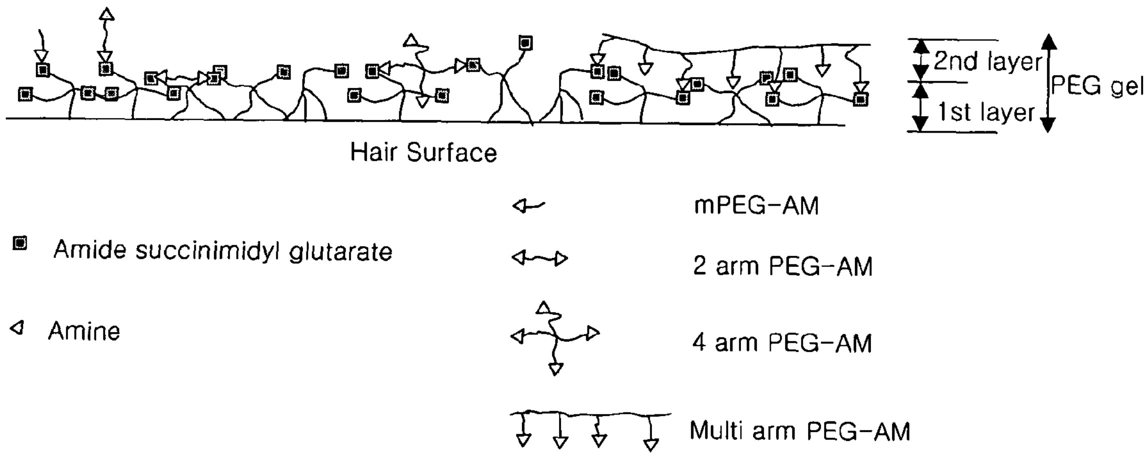
2nd layer
1st layer
PEG gel
Hair Surface
Amide succinimidyl glutarate
Amine
mPEG-AM
2 arm PEG-AM
4 arm PEG-AM
Multi arm PEG-AM

Fig. 3

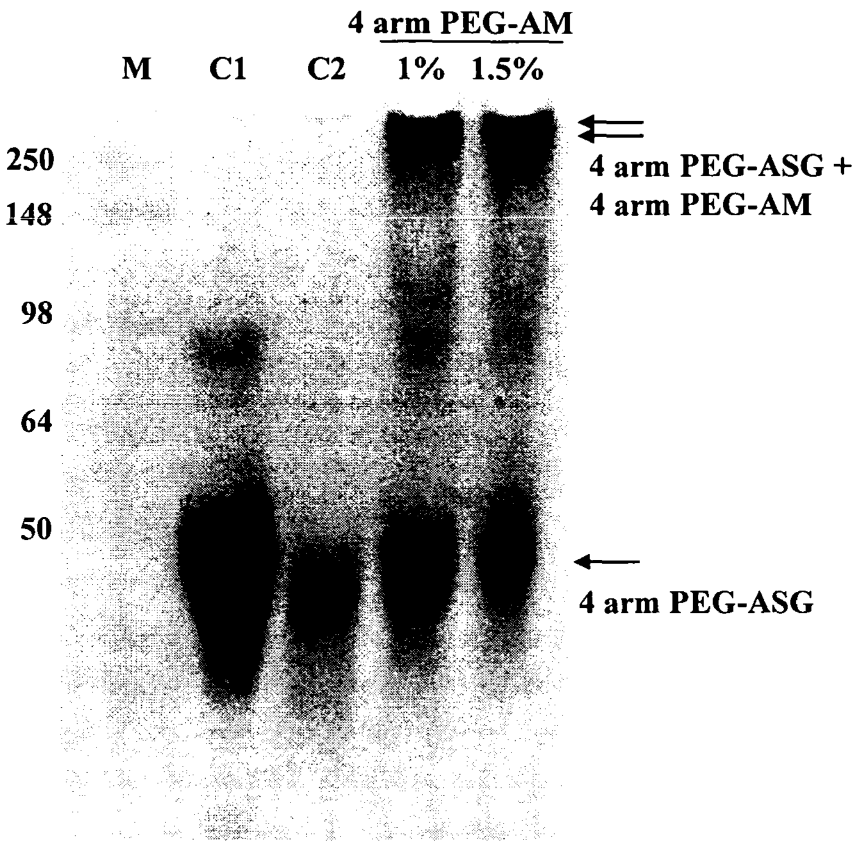


C1 : 4 arm PEG-ASG
C2 : 4 arm PEG-AM
1% : 5% 4 arm PEG-ASG + 1% 4 arm PEG-AM
1..5% : 5% 4 arm PEG-ASG + 1.5% 4 arm PEG-AM

Fig. 4

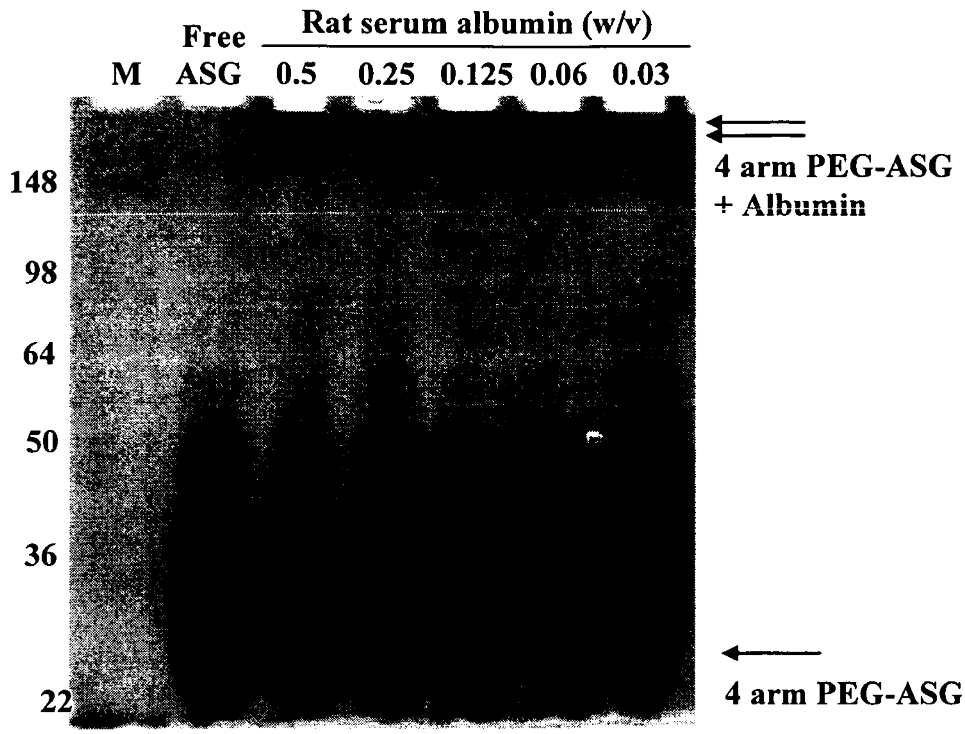
Free
Rat serum albumin (w/v)
M
ASG
0.5
0.25
0.125
0.06
0.03
148
98
64
50
36
22
4 arm PEG-ASG
+ Albumin
4 arm PEG-ASG

Fig. 5

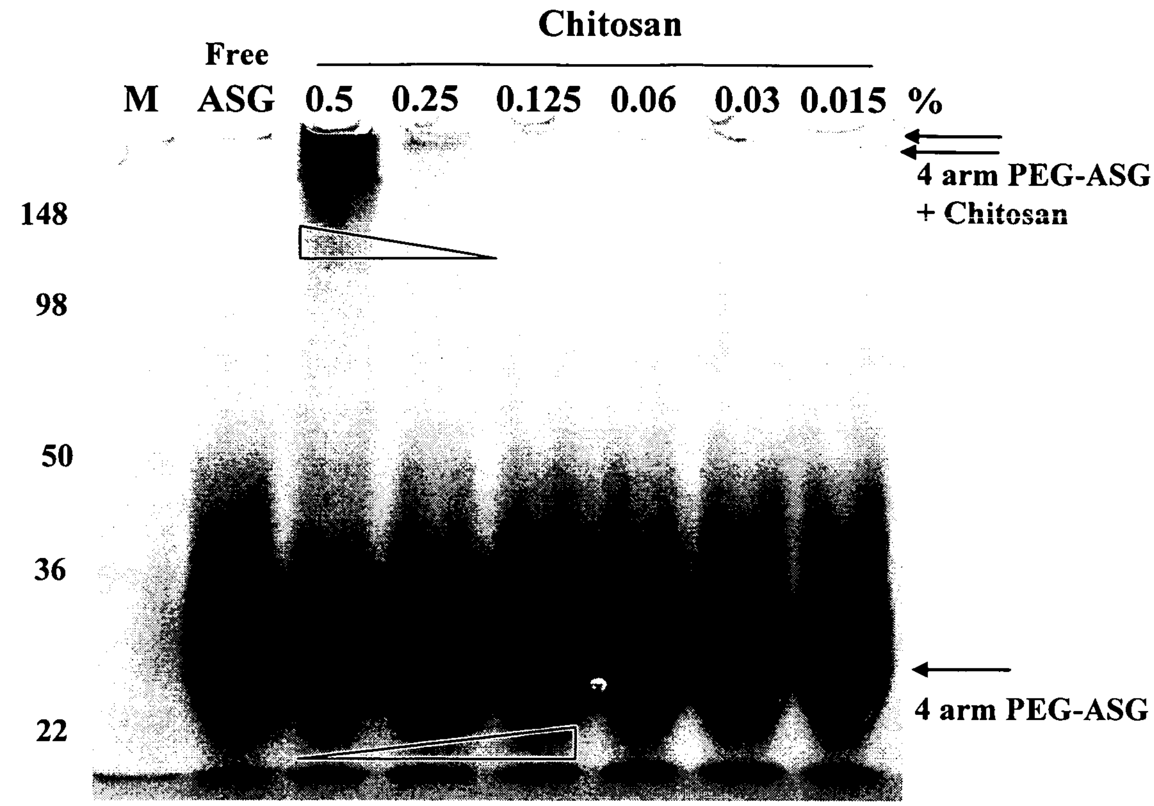
Chitosan
Free
M
ASG
0.5
0.25
0.125
0.06
0.03
0.015
%
4 arm PEG-ASG
+ Chitosan
148
98
50
36
22
4 arm PEG-ASG

Fig. 6

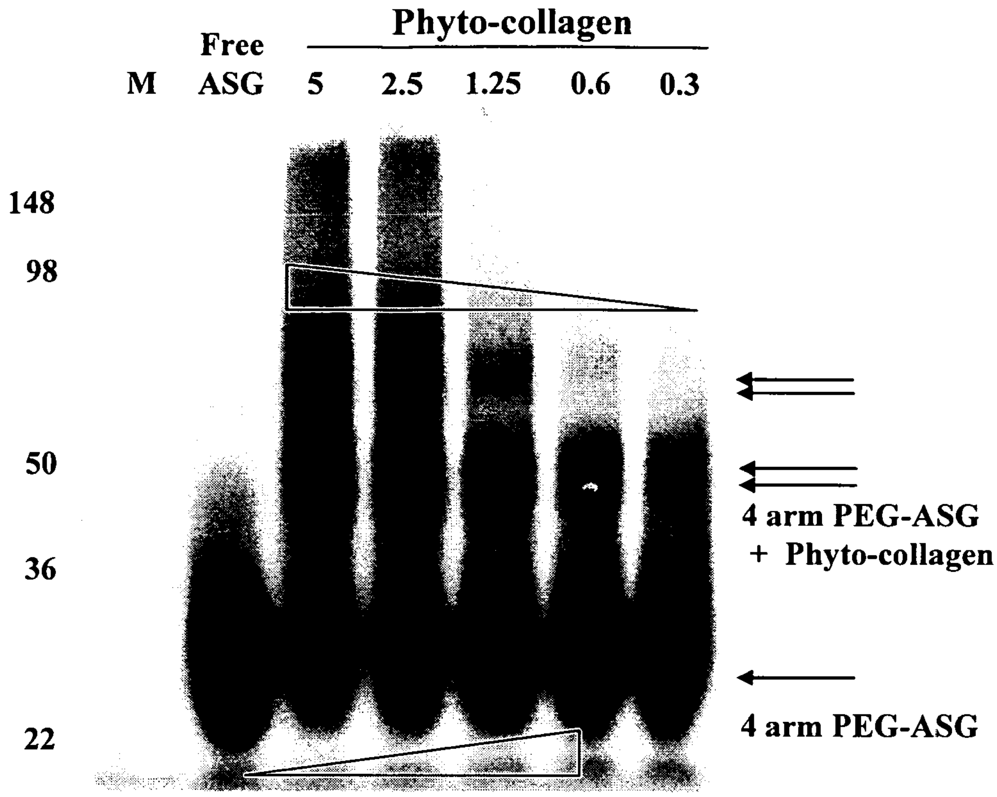
Phyto-collagen
M
Free ASG
5
2.5
1.25
0.6
0.3
148
98
50
36
22
4 arm PEG-ASG + Phyto-collagen
4 arm PEG-ASG

Fig. 7

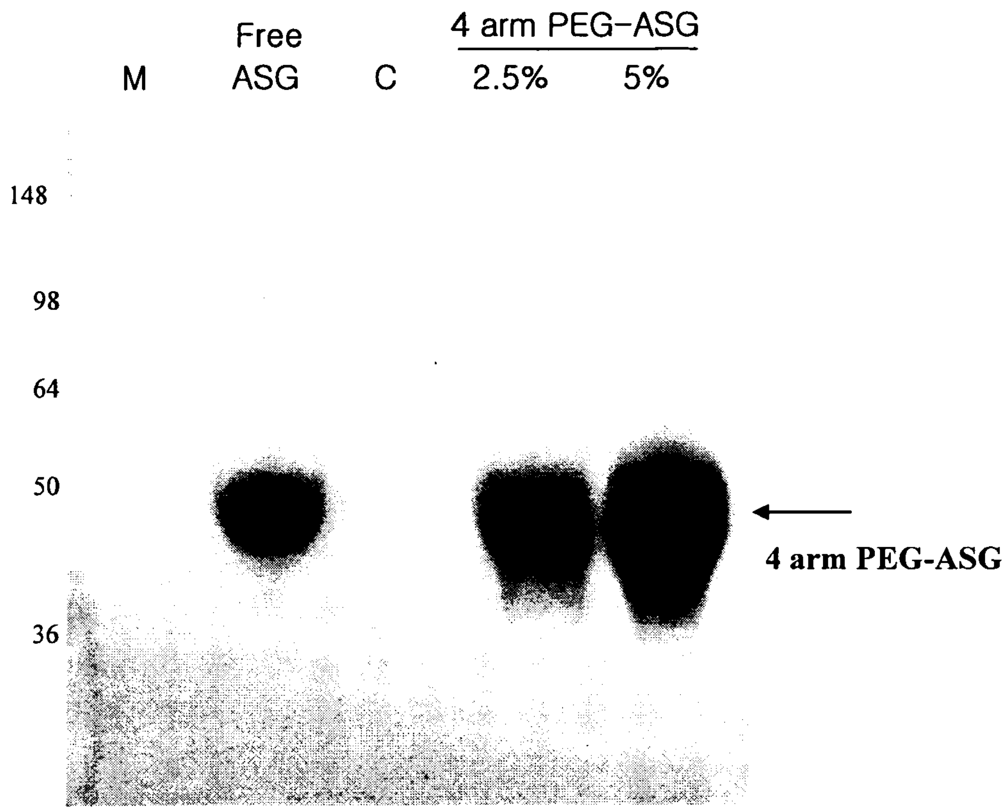

Fig. 8

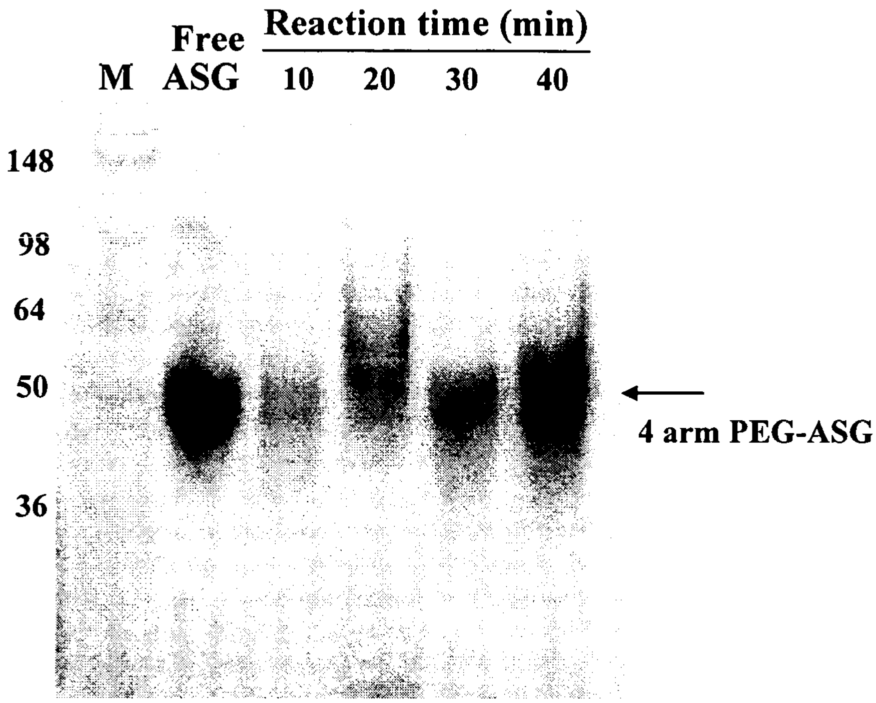
Free
Reaction time (min)
M ASG 10 20 30 40
148
98
64
50
36
4 arm PEG-ASG

Fig. 9

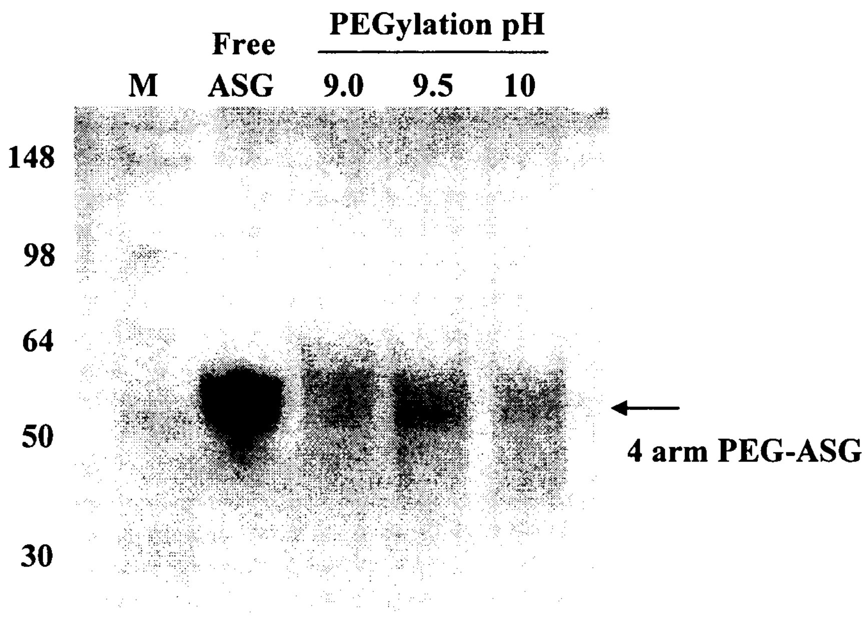
Free
PEGylation pH
M ASG 9.0 9.5 10
148
98
64
50
30
4 arm PEG-ASG

Fig. 10

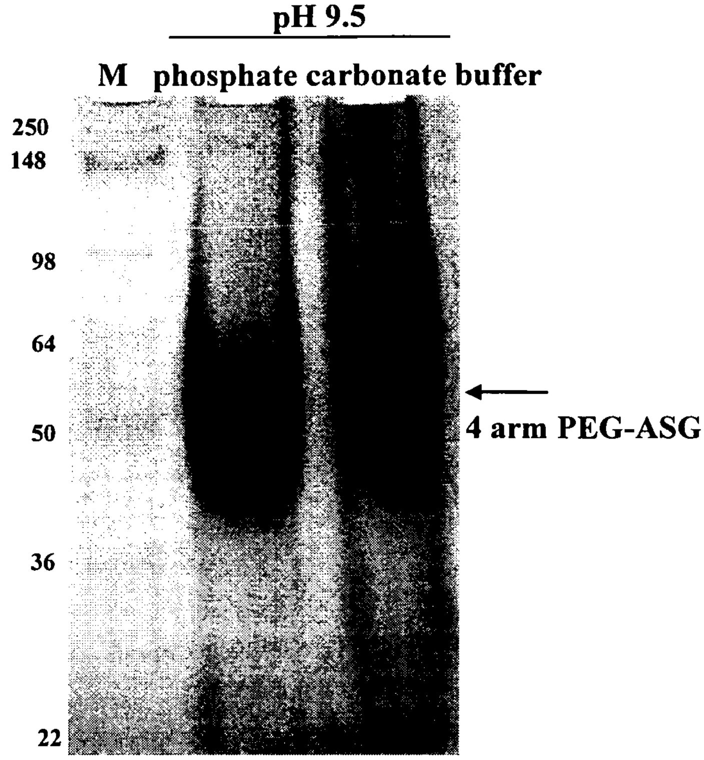
pH 9.5
M
phosphate
carbonate buffer
250
148
98
64
50
36
22
4 arm PEG-ASG

Fig. 11

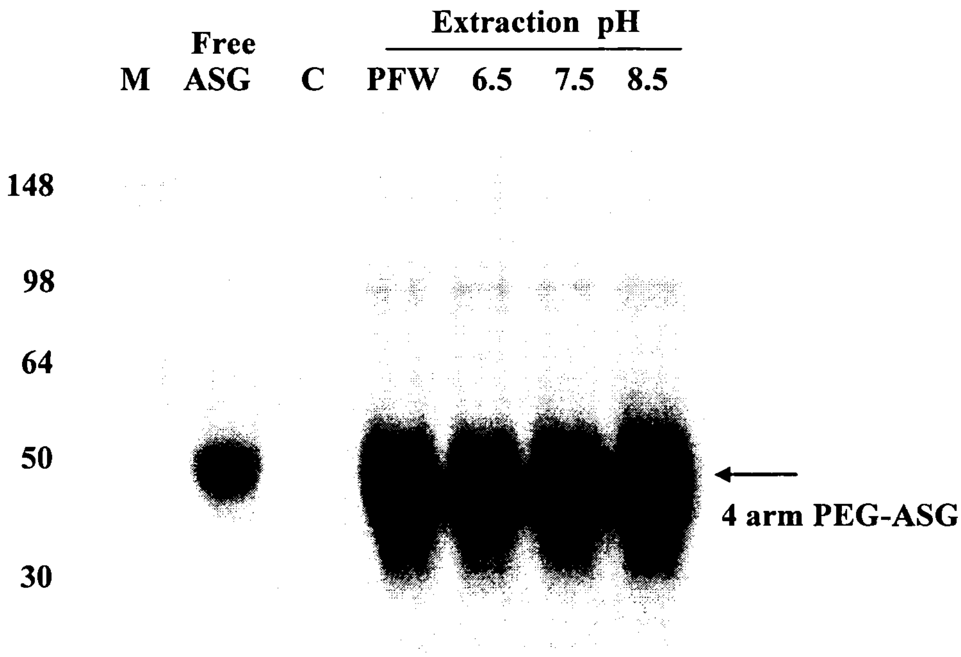
Extraction pH
M
Free ASG
C
PFW
6.5
7.5
8.5
148
98
64
50
30
4 arm PEG-ASG

Fig. 12

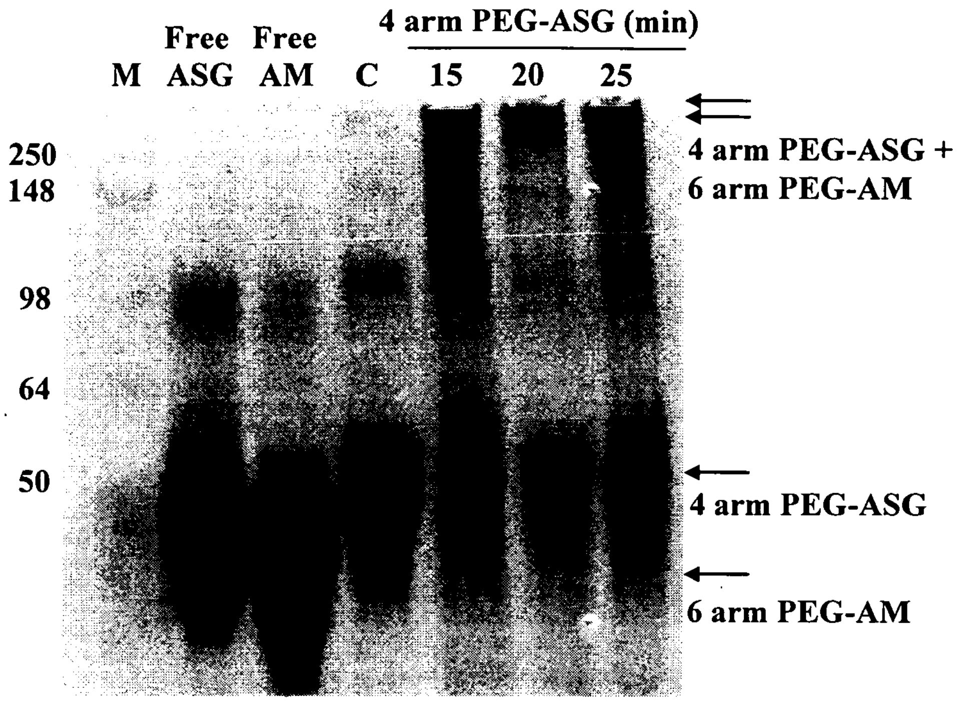


C: Only 4 arm PEG-ASG, 40 min

15: 5% 4 arm PEG-ASG, 15min -> 5% 4 arm PEG-AM, 30min

20: 5% 4 arm PEG-ASG, 20min -> 5% 4 arm PEG-AM, 30min

25: 5% 4 arm PEG-ASG, 25min -> 5% 4 arm PEG-AM, 30min

Fig. 13

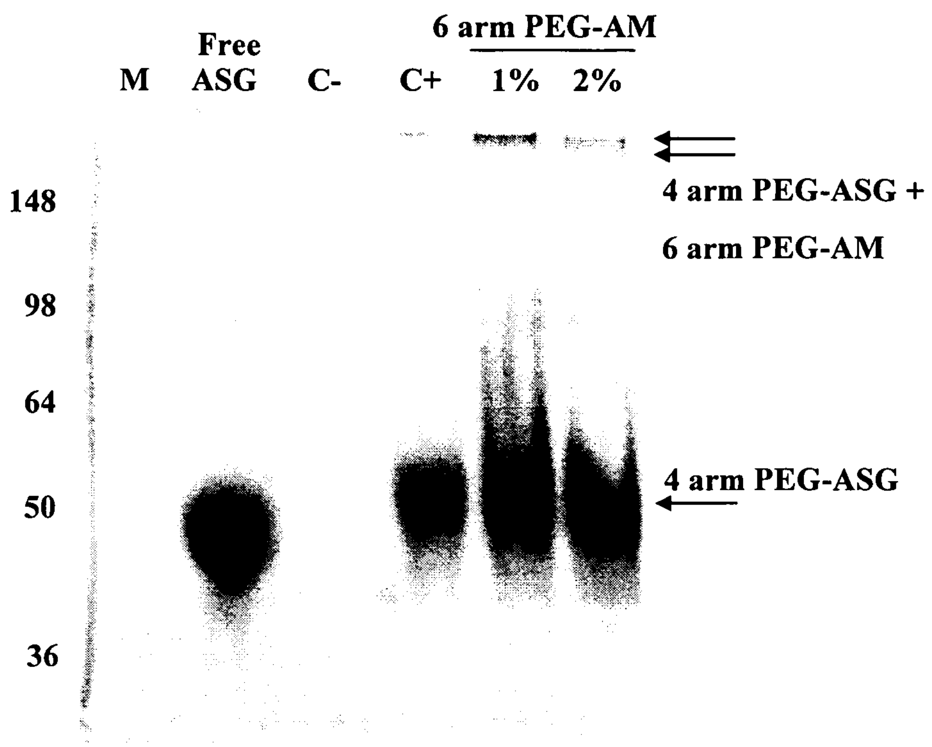


Free ASG : 4 arm PEG-ASG 20 k
C- : Non pegylation
C+ : only 4 arm PEG-ASG, 40 min
1% : 5% 4 arm PEG-ASG, 40 min -> 1% 6 arm PEG-AM, 30 min
2% : 5% 4 arm PEG-ASG, 40 min -> 2% 6 arm PEG-AM, 30 min

Fig. 14

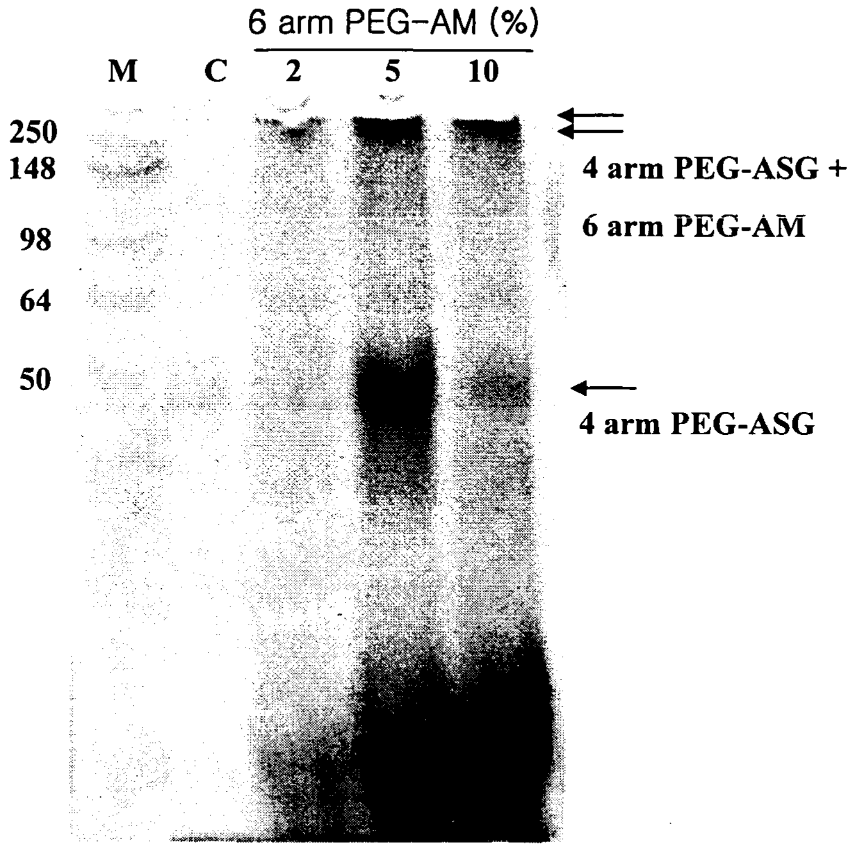


**C: Only 4 arm PEG-ASG, 40 min**

**2: 5% 4 arm PEG-ASG, 40min -> 2% 4arm-AM, 30 min**

**5: 5% 4 arm PEG-ASG, 40min -> 5% 4arm-AM, 30 min**

**10: 5% 4 arm PEG-ASG, 40min -> 10% 4arm-AM, 30 min**

Fig. 15

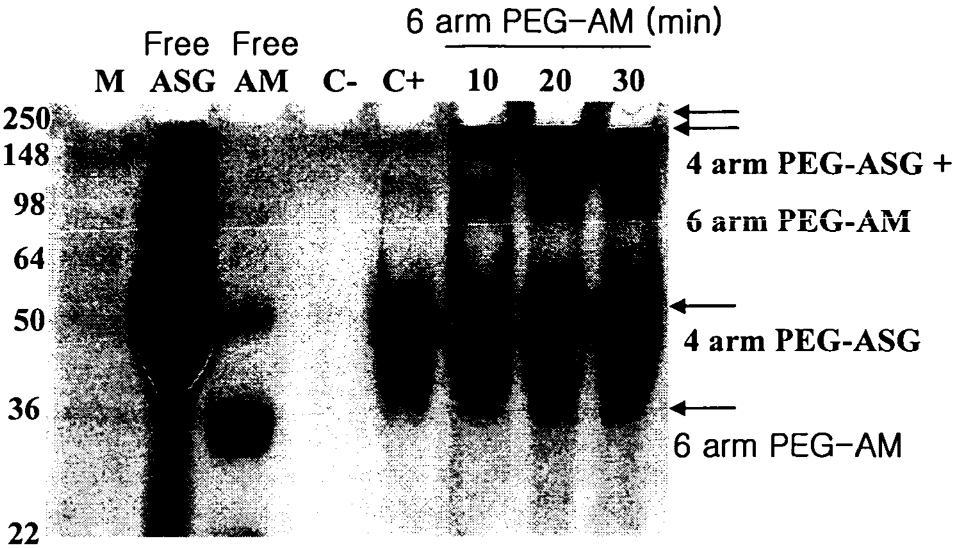


Free ASG: 20k 4 arm PEG-ASG
Free AM: 10k 6 arm PEG-AM
C- : Non-pegylation
C+: 5% 4 arm PEG-ASG, 40 min
10 : 5% 4 arm PEG-ASG, 40 min -> 5% 6 arm PEG-AM, 10 min
20 : 5% 4 arm PEG-ASG, 40 min -> 5% 6 arm PEG-AM, 20 min
30 : 5% 4 arm PEG-ASG, 40 min -> 5% 6 arm PEG-AM, 30 min

Fig. 16

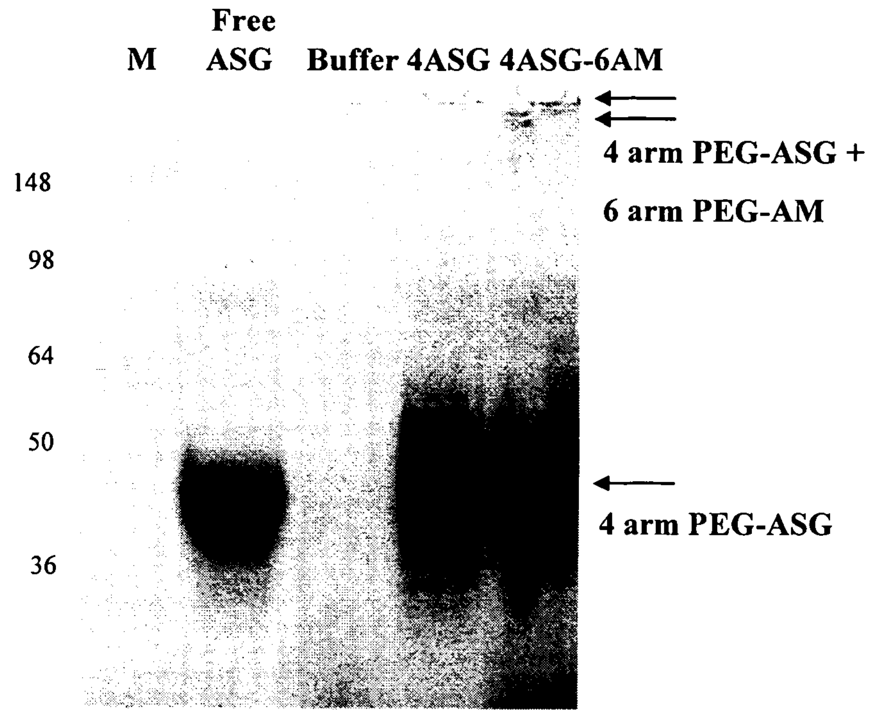


Free ASG : 20k 4 arm PEG-ASG

Buffer : Non-pegylation

4ASG: Only 4 arm PEG-ASG, 40 min

4ASG-6AM : 5%4 arm PEG-ASG, 30min -> 5% 4arm-AM, 30 min

Fig. 17

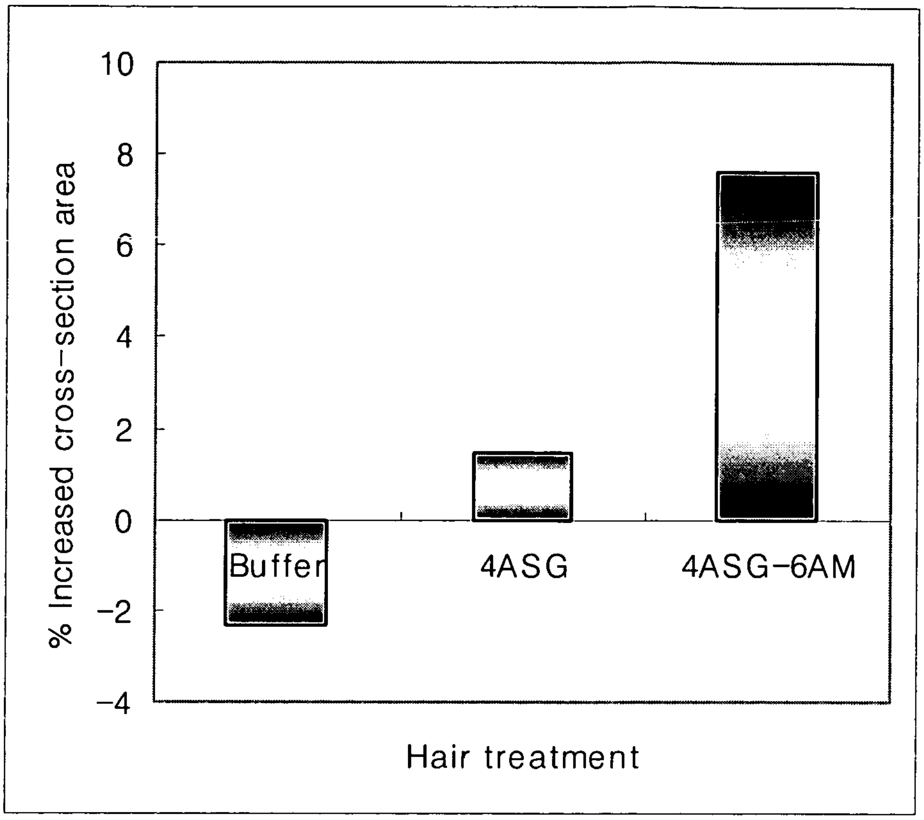
10
8
6
4
2
0
-2
-4
% Increased cross-section area
Buffer
4ASG
4ASG-6AM
Hair treatment

Fig. 18

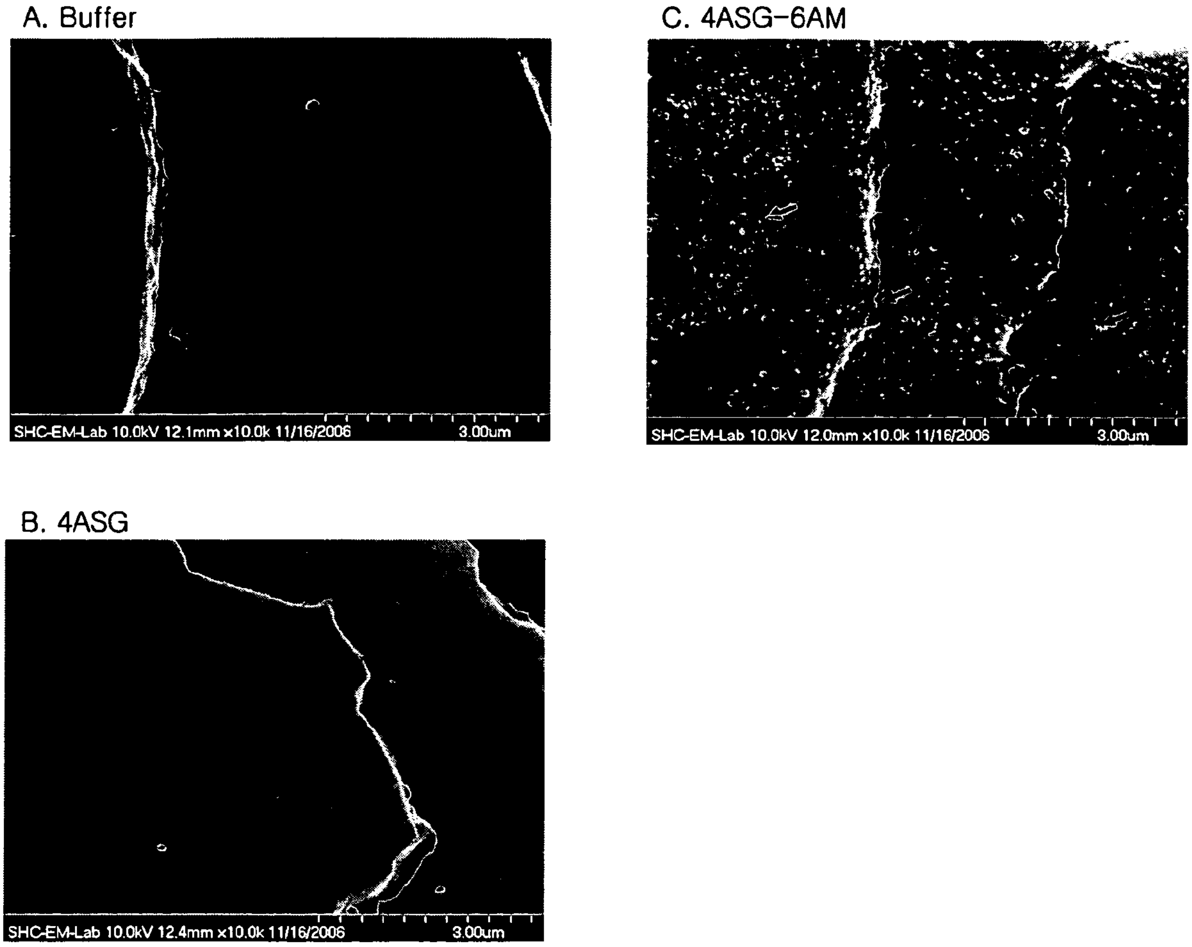
A. Buffer
SHC-EM-Lab 10.0kV 12.1mm x10.0k 11/16/2006
3.00um
C. 4ASG-6AM
SHC-EM-Lab 10.0kV 12.0mm x10.0k 11/16/2006
3.00um
B. 4ASG
SHC-EM-Lab 10.0kV 12.4mm x10.0k 11/16/2006
3.00um

Fig. 19

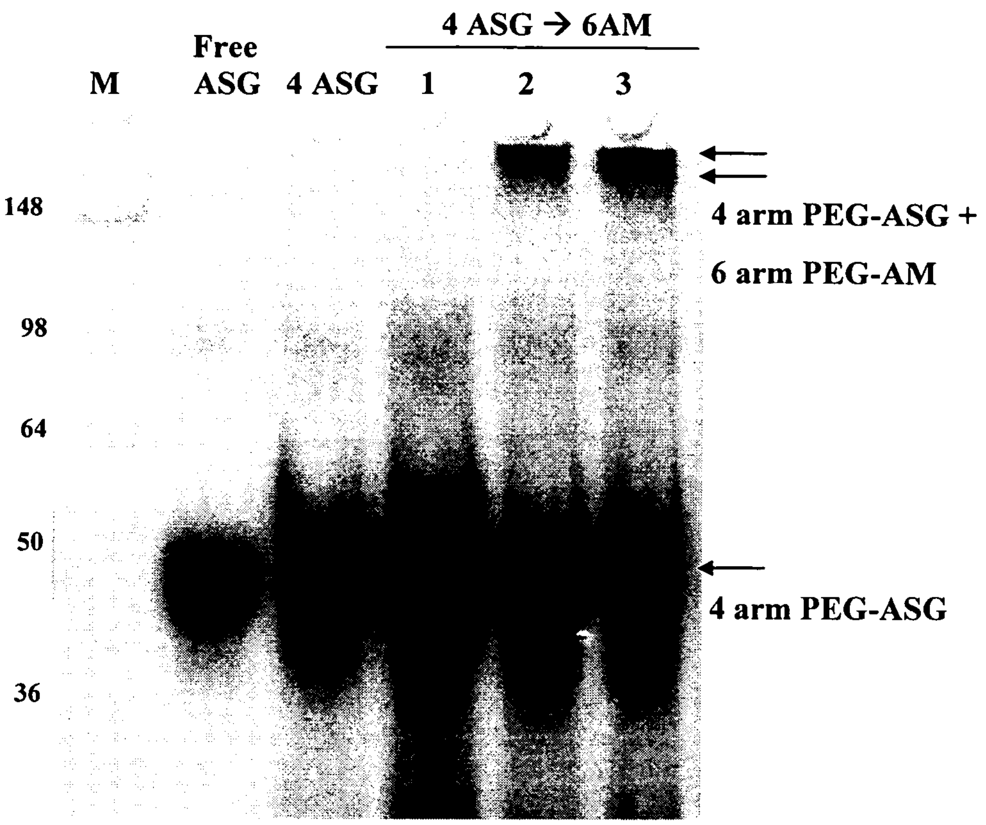


Free ASG : 20 K P4ASG (C)
4ASG : only 4 arm PEG-ASG
1 : 4 arm PEG-ASG-> 6 arm PEG-AM one time
2 : 4 arm PEG-ASG-> 6 arm PEG-AM two times
3 : 4 arm PEG-ASG-> 6 arm PEG-AM three times

Fig. 20

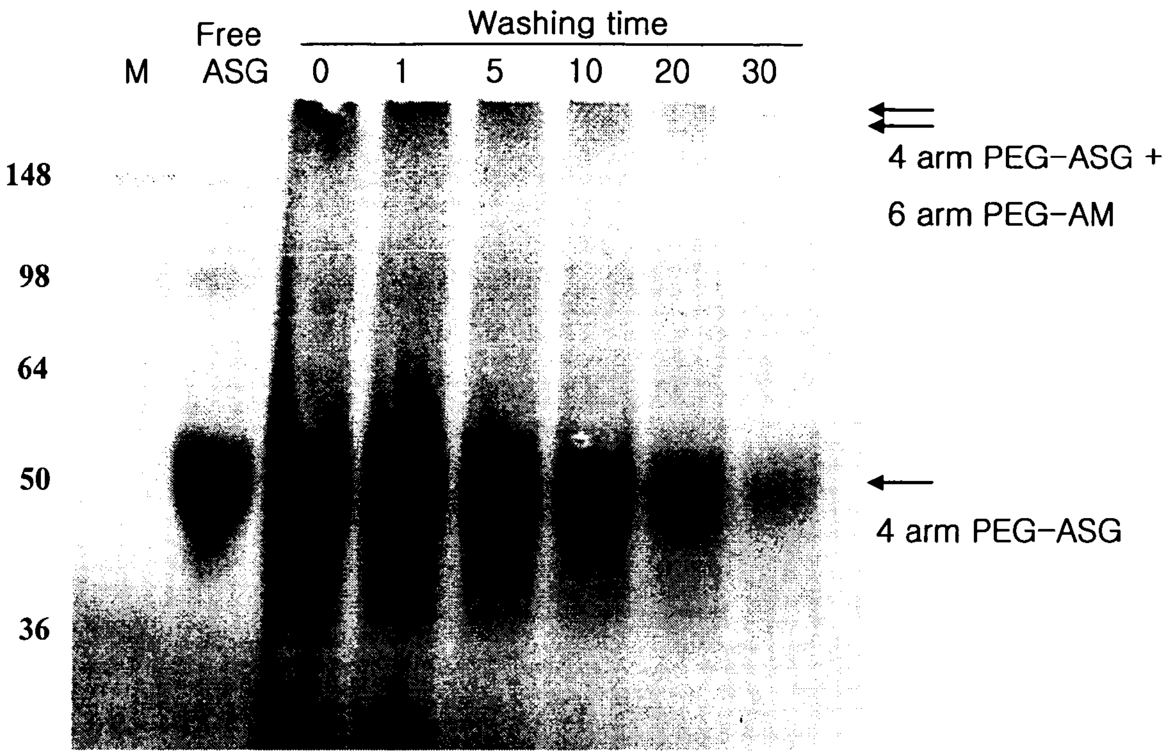
Washing time
M
Free ASG
0
1
5
10
20
30
148
98
64
50
36
4 arm PEG-ASG +
6 arm PEG-AM
4 arm PEG-ASG

Fig. 21

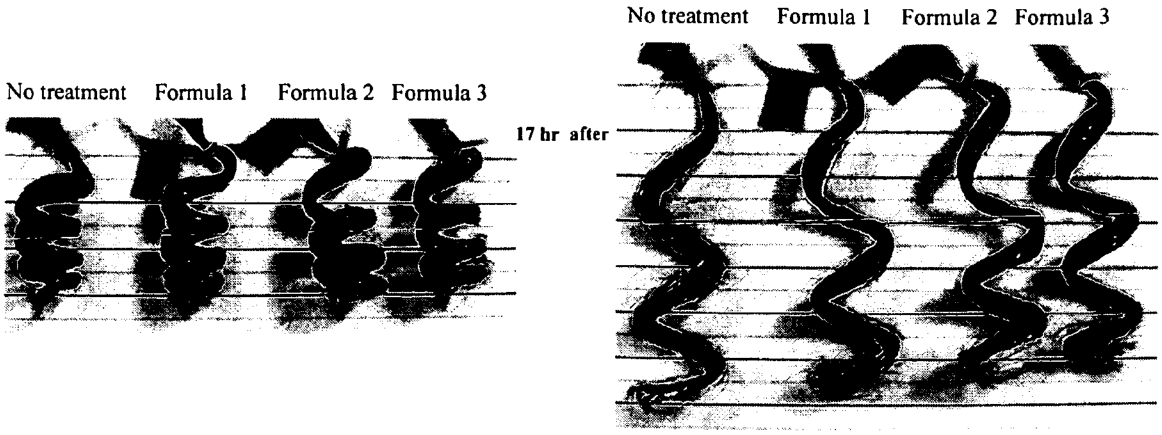
No treatment Formula 1 Formula 2 Formula 3
17 hr after
No treatment Formula 1 Formula 2 Formula 3

Fig. 22

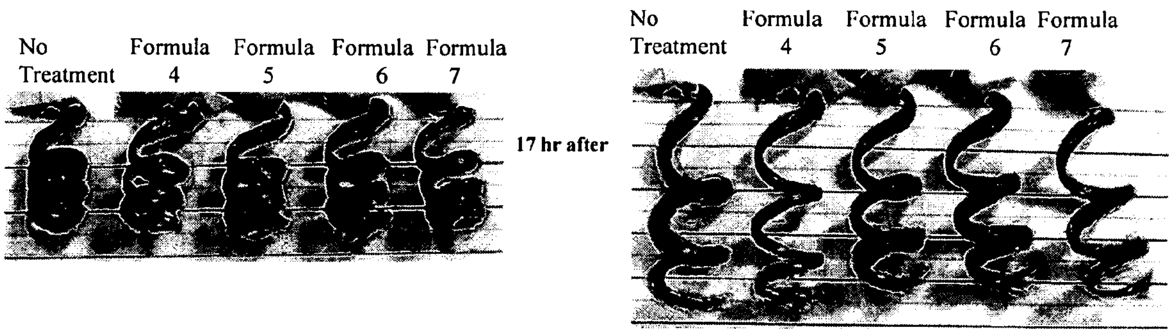
No Treatment Formula 4 Formula 5 Formula 6 Formula 7
17 hr after
No Treatment Formula 4 Formula 5 Formula 6 Formula 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020060130568
- KR 1020070045253
- US 6447803 B, Sorrentino
- US 7048916 B
- US 6548051 B, Rollat
- US 6410005 B, Gallengullos
- US 6436412 B, Quinn
- US 6258347 B, Sakuta